(19)

Europäisches
Patentamt
European
Patent Office
Office européen
des brevets

(11) **EP 4 529 857 A2**

(12) **EUROPEAN PATENT APPLICATION**

(43) Date of publication:
**02.04.2025 Bulletin 2025/14**

(21) Application number: **24204863.5**

(22) Date of filing: **19.02.2021**

(51) International Patent Classification (IPC):
***A61B 8/08*** (2006.01)

(52) Cooperative Patent Classification (CPC):
**A61B 8/4245; A61B 8/42; A61B 8/4254;**
**A61B 8/4455; A61B 8/4477; A61B 8/4488;**
**A61B 8/4494; A61B 8/5207; A61B 8/5269;**
**A61B 8/58;** A61B 8/0883; A61B 8/4209;
A61B 8/4236

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **18.03.2020 EP 20164030**

(62) Document number(s) of the earlier application(s) in
accordance with Art. 76 EPC:
**21158222.6 / 3 881 770**

(71) Applicant: **Pulsify Medical**
**3001 Leuven (BE)**

(72) Inventors:
• **Barbosa, Daniel**
**4710-089 Braga (PT)**
• **Moraes, Rodrigo**
**3000 Leuven (BE)**

• **Stoffels, Steve**
**3053 Haasrode (BE)**
• **D'hooge, Jan**
**2800 Mechelen (BE)**

(74) Representative: **DTS Patent- und Rechtsanwälte**
**PartmbB**
**Brienner Straße 1**
**80333 München (DE)**

Remarks:
•This application was filed on 06-10-2024 as a
divisional application to the application mentioned
under INID code 62.
•Claims filed after the date of filing of the application /
after the date of receipt of the divisional application
(Rule 68(4) EPC).

(54) **ULTRASOUND SYSTEM**

(57)     The invention relates to an ultrasound system
(1), in particular for cardiac monitoring, comprising a
transducer array (3) with a plurality of transducer array
elements (3a), a control unit (40), which is coupled to the
transducer array (3), for controlling the transducer array
(3), and a data processing unit (41) for receiving data
from the transducer array (3) and processing the received data. Therein, the ultrasound system (1) further
comprises a deformation correction module (43), which is
configured to perform a deformation correction step,
wherein deformation information is determined for the
transducer array (3). The invention also relates to a
method for operating an ultrasound system (1), wherein
a deformation correction step is performed, wherein deformation information is determined for the transducer
array (3).

Fig. 1

EP 4 529 857 A2

**Description**

The present invention relates to an ultrasound system, in particular for cardiac monitoring.

Wearable ultrasound monitor systems monitor cardiac function over time. The ultrasound monitor can be body worn in a patch type format.

US 2016/0136462 A1 discloses a wearable ultrasound device and method of using the device including a power controller with a power source and at least one integrated circuit that delivers electrical power to an applicator. The applicator is electrically coupled to the power controller and a surface of the applicator transmits ultrasound to a wearer for a given duration. The applicator includes radio frequency (RF) drive electronics, an ultrasound transducer coupled to the drive electronics, a monitoring apparatus that includes a thermal cutoff coupled to the drive electronics.

WO 2018/102828A1 discloses an ultrasound coupling patch for use with ultrasound transducers, and more particularly to ultrasound coupling patches having a gel capture feature.

WO 2019/162485 A1 discloses a method and an apparatus for performing ultrasound measurements of a propagation medium containing non uniformities, with a transmitter/transducer with a plurality of transmitting elements for emitting ultrasound excitation pressure waves.

In ultrasound systems with a large number of ultrasound transducer elements, e.g., when a transducer array with 10.000 or 100.000 elements is used, individually sampling every single transducer element leads to massive amounts of data. The system needs accordingly high capabilities for the throughput and the processing of this data. A high amount of power is required to drive and read such an array and all its individual transducer elements.

To reduce the overall data-rate and the power consumption while maintaining sufficient image quality for the respective application, the system complexity needs to be taken into account.

While flexible ultrasound probes have already been exploited for non-destructive testing (NDT) of industrial products with complex surface, the use of such conformal probes in medical applications is still in its infancy. One reason for this is that the surface shape of a probe is difficult to estimate, and a higher image complexity needs to be treated in medical data compared to internal structure of industrial products. Considering that precise information of an element's position is required for, e.g., accurate image reconstruction via delay- and-sum beam-forming, methods to robustly estimate the shape of a transducer array are necessary.

Also, if a flexible ultrasound device is used, it usually has an unknown form factor, e.g., after fixing the device to a patient's body. This creates issues, e.g., for beam-forming and image reconstruction algorithms, since these depend on "knowing" the exact positions and orientations of the individual transducer elements with respect to each other. If these positions and orientations are unknown, the beam-forming delays cannot be calculated exactly and consequently the beam profile will not be optimal. Also, image reconstruction based on incorrect positional information will lead to a deformed and/or un-sharp image, i.e. an image of suboptimal quality.

It is the goal of the current invention to provide an ultrasound system and a method to operate the ultrasound system, wherein appropriate corrections can be performed without "knowing" the device's shape a priori.

This problem is solved by an ultrasound system according to claim 1 of the attached set of claims. Further advantageous embodiments are given in the dependent claims.

The ultrasound system, in particular for cardiac monitoring, comprises a transducer array with a plurality of transducer array elements, a control unit, which is coupled to the transducer array, for controlling the transducer array, and a data processing unit for receiving data from the transducer array and processing the received data.

The system further comprises a deformation correction module, which is configured to perform a deformation correction step wherein the measured data e.g. ultrasound signals and/or images are corrected for deformation induced distortions in the data. The deformation can be caused e.g. by conforming the array to a curved and/or freeform surface. This conformation will cause the patch to deform from its reference shape such that the deformed array will tend to follow the freeform surface as close as possible. The reference shape of the transducer could e.g. be a flat position.

Conformation is to be understood in this sense especially as the act of deforming ultrasound array to follow a freeform surface as close as possible. The act of conformation leads to a temporary deformation, i.e. a temporary spatial position and/or pose and/or shape and/or arrangement in space of the individual elements of the systems, especially parts of the system such as the transducer array and/or the flexible patch with the array. Deformation correction can also be understood as a step to correct the measured ultrasound signal and/or images for artifacts introduced due to the deformation of the ultrasound array with respect to it's reference shape (which is in the unused or normal state flat or substantially flat) or change in shape. Based on these corrected data, the final results can be calculated and computed with substantially enhanced accuracy.

Thus, the system advantageously allows using the ultrasound system very flexibly, even under conditions where a deformation correction is needed to perform accurate ultrasound measurements and imaging. With a deformation correction the system corrects the measured ultrasound signal and/or images for artifacts introduced due to the deformation of the ultrasound array with respect to its reference shape. This is then used for better accuracy in measurements.

The invention relates to correction of the measured ultrasound signal and/or images for artifacts introduced due to the deformation of the ultrasound array with respect to its reference shape. This deformation correction can be done by the reconstruction of the unknown shape (i.e. deformation) of a transducer array, based on measurements performed by the uncalibrated transducer array and the images derived from those measurements. The information on the deformed shape can be used to correct the artifacts in the ultrasound signal and/or images. Additionally or alternatively, instead of finding the deformed shape of the the transducer array or the patch, the current invention also includes a correction for the deformed shape of the transducer array without needing to reconstruct the deformation of the transducer array, or knowing this deformation *a priori.*

The invention is based on the idea that, by performing measurements with the system under operation conditions, deformation information may be deduced that allows correcting for a deformation of the transducer array relative to a predetermined reference shape (e.g. the flat initial configuration) of the array. For example, deviations from a regular, flat and even arrangement of the transducer array (e.g. a so-called "reference shape") is assumed in the beginning and the subsequent deformation correction is applied based on deviations of measured data from the expected values. An alternative method of the invention applies this deformation correction without knowing or determining the deformed shape.

In particular, the ultrasound system can be in general an ultrasound system as disclosed by WO 2019/162485 A1. The further disclosed features can be added for example to the system as disclosed in WO 2019/162485 A1.

The system may be configured for cardiac monitoring, which may for example comprise monitoring of cardiac activity and/or cardiac properties of a patient. Monitoring cardiac activity may comprise monitoring of parameters such as cardiac rhythm and/or cardiac output.

The system has at least one transducer array, i.e., a device or unit comprising a plurality of transducer array elements. In particular, the transducer array may have a chip design. The transducer array elements are units, which may be used as an actuator for generating an ultrasound pulse or signal. Generally speaking, one transducer array element may consist of one or more transducer sub-elements. A single transducer can be a piezoelectric micro-machined ultrasonic transducer (pMUT) component or a capacitive micro-machined ultrasonic transducer (cMUT) component.

Different techniques may be used to manufacture transducer elements and/or sub-elements. For example, cMUT units may be fabricated using a polymer-based technique, such as described by Gerardo et al. in "Fabrication and testing of polymer-based capacitive micro-machined ultrasound transducers for medical imaging", (Microsystems & Nanoengineering (2018) 4:19).

In order to reduce the complexity of the system, several transducer elements may be combined into a functional unit and treated as a single "element", i.e., the combined transducer array elements are driven at that same time as if they are a single unit.

The transducer array elements in the transducer array can be connected in series or parallel, such that they can be driven optimally.

In an embodiment of the system, the transducer array is flexible. Thus, the system can advantageously adjust its geometry to a probe or sample surface, e.g., a patient's body. In particular, the transducer array elements may be flexibly oriented towards each other.

For example, the transducer array elements may be supported by a flexible substrate such that they are arranged on a flat or curved plane. The substrate may, e.g., comprise a polymer material.

Thus, the array can assume different shapes, wherein the position and/or orientation and/or pose of an individual transducer array element relative to another transducer array element and/or relative to the transducer array as a whole is deviating from a predetermined or expected position or pose. For example, the transducer array may be a bent one or several transducer array elements may be tilted with respect to others.

To determine the actual form factor and/or shape of the transducer array, different methods may be used. For example, a strain sensor may integrated in the transducer in order to determine the shape.

In an embodiment of the system, the transducer array is integrated into an adhesive patch for fixing the transducer array to a patient's body. The system can then advantageously be easily used, e.g., for monitoring a patient's body functions, such as cardiac functions, especially for long-term monitoring.

The transducer array may be integrated into the patch by providing it with an adhesive area, which is suited for attaching the patch to a patient's body surface. Also, a textile or other flexible material may be provided with an adhesive material or simply a mechanical strap without adhesive or a combination thereof and used to fix the transducer array to the body surface. In particular, the patch may be configured such that it secures the transducer array in close proximity to the body surface.

Also, the ultrasound system comprises a control unit. The control unit may comprise several components, which may be structurally integrated with each other, for example in a common casing or envelope structure, or which may be implemented as structurally separated components, which may be coupled to each other.

The transducer array elements are configured to generate and/or receive ultrasound, in particular ultrasound pulses or signals, based on a control signal. To provide the respective control signals for the transducer array, the control unit may comprise a send module and a receive module, which may be implemented in a driver module for the transducer array and/or for transistors, which may control the transducer array elements.

The control unit may be configured to perform different tasks such as, but not limited to, the following examples: The control unit may be configured to perform digital signal processing and/or filtering. Also, the control unit may be configured to perform operations related to storing and/or retrieving information, in particular in a memory module, such as a random access memory (RAM) module. Also, the control unit may be configured to perform operations for driving an external interface, such as an interface to a memory module, a transceiver unit, e.g., via a USB interface or a wireless interface, and/or receiving control signals from other units. The control unit may also perform operations for controlling the transducer array, e.g., through a switch linked to a transducer array element, such as a switch can be a transistor or more specifically a thin-film-transistor (TFT) element, or a matrix of such switches/transistors, e.g., in a TFT matrix. The TFT matrix can be a(n) (integral) part in terms of function and/or structure of the control unit. Also, the control unit may be configured to control a TFT element connected to a transducer element,, to generate delays for generating and/or sensing ultrasound pulses and signals, to define generated ultrasound pulses, beam-forming operations, and/or to control power management. Each of said functions may be carried out and/or implemented in an ASIC design individually or in combination. Also, these functions may be implemented as modules of the control unit.

The control unit may comprise a switch e.g. a transistor or thin-film transistor (TFT), which is configured to control a transducer array element. In particular, an array of switches e.g. thin-film transistors may be provided, which is coupled to the transducer array.

In particular, the TFT switch array (e.g. TFT array) may be structurally integrated with the transducer array in one component, e.g., in a common casing or envelope structure.

The control unit may consist of or comprise one or several microprocessors. Also, the control unit may comprise an application-specific integrated circuit (ASIC) design. The control unit, in particular a microprocessor of the control unit, may be fully or partially integrated into a printed circuit board (PCB).

The system may comprise a field programmable gate array (FPGA) and/or a digital application-specific integrated circuit (ASIC), configured such that one or more control algorithms for the transducer array are carried out. The FPGA and/or ASIC may be comprised by the control unit.

In an embodiment, the system further comprises a beam-former module. Herein, the beam-former module is configured to apply a time delay to at least one transducer array element or a plurality of transducer array elements. This allows advantageously easy control for high-resolution measurements.

In particular, the time delay may be applied, when the transducer array element or the plurality of transducer array elements is used for sending ultrasound pulses and/or receiving ultrasound signals.

The time delay for a transducer array element or a plurality of transducer array elements may be defined relative to controlling other transducer array elements.

Also, the beam-former module and/or another unit of the system may be utilized to determine the delay. In particular, the delay is determined for each transducer array element or for a plurality of transducer array elements, to which the determined delay is to be applied.

In particular, the control unit may comprise an ASIC for implementing a beam-former control, and/or a design for a signal processing algorithm, e.g., for applying spatial and/or temporal sparsity conditions for sending ultrasound pulses and/or for collecting or providing data.

In particular, the beam-former control can for example comprise an electronic delay and sum circuit that can apply different delays to a certain number of transducer array elements, for example 8 to 32, e.g. 14, 15, 16, 17 or 18 transducer array elements. or functional units. It may sum the output of these channels into one single output value. In particular, by applying specific delays to each functional unit of transducer array elements, an ultrasound pulse may be generated in a coordinated fashion such that a specific profile for the ultrasound beam is obtained. Also, elements receiving ultrasound signals may be controlled by a transmit beam-former in a coordinated way to provide targeted measurements inside a sample volume.

The data processing unit may be implemented in different ways. It may for example comprise a microprocessor. In particular, it may comprise an ASIC design. Also, the data processing unit and the control unit may be implemented as software modules that are run, at least partially, on a common computational unit. For example, a processing unit may read from a memory and execute instructions for performing operations to implement the data processing unit and/or control unit.

Also, the data processing unit may comprise one or several analog-digital converters (ADC) and/or digital-analog converters (DAC). These converters are configured to link analog and digital channels to each other and allow both digital data collection and treatment, and treating analog control and detection signals. A time gain controller may be implemented using a DAC element and/or a different architecture.

The complexity reduction module may be implemented as software module, comprising instructions for computational operations that implement the function of complexity reduction. This software module may in particular be implemented as a module of the control unit. Specifically, the complexity reduction module may be comprised, at least partially, by the control unit and/or by the data processing unit.

The complexity reduction module implements functions to reduce data and sensor complexity and/or quantity. Such functions may impact the generation and/or the detection of ultrasound pulses and signals by the transducer array, or they may influence the data acquisition and/or treatment.

The coupling between the transducer array and the control unit may comprise a wire connection, in particular an interface for data and/or power transmission between the transducer array and the control unit. In particular, the control unit and/or a part of the control unit may be arranged on the sensor array or may be integrated with the sensor array into one common part. The sensor array can be embodied especially by means of the combination of a switch (e.g. TFT) array and a transducer array.

In particular, there is a wire connection and/or a wireless interface provided, if the control unit controls the sensor array. If a wireless sensor array is implemented, the control unit may be arranged on the sensor array and/or integrated into one component with the sensor array.

In particular, if the sensor array is connected to another element by a wire, at least part of the controlling of the sensor array may be implemented on the sensor array and/or integrated into one component with the sensor array. Also, an additional control unit may be provided, e.g., in a separate PC or control unit, when there is access through wires to the sensor array. A sensor array is for example a combination of a transducer array and a TFT array. Parts of the controller can be integrated on a TFT array by an integrated circuit design or by bonding ICs to the TFT.

In an embodiment, the ultrasound system further comprises a power source unit for providing energy to the transducer array and/or the control unit. The system can thus be advantageously used in a very flexible manner, in particular when a portable power source is provided. For example, the power source may comprise a battery, which is integrated in a portable device as a component of the system.

In an embodiment, at least one sub-array of transducer array elements is defined and controlled as a functional unit for sending ultrasound pulses and/or receiving ultrasound signals.

Rather than operating the transducer array as a single whole and using each element as an individual unit, a sub-array scanning architecture may be used, i.e., groups of transducer array elements are combined to functional units. In particular, neighboring elements are combined.

In particular, a control algorithm may be provided for controlling each sub-array within the transducer array. The control algorithm may control, for each sub-array, a location of the sub-array within the plurality of transducer array elements. Also, the control algorithm may control, for each sub-array, a focus depth and/or a steering angle for an ultrasound sending beam profile and/or an ultrasound receiving beam profile implemented by the sub-array of transducer array elements; such a beam-forming algorithm may be implemented as a software module for a programmable unit and/or by chip design, e.g., in an ASIC.

When individual sub-arrays are controlled as functional units for sending ultrasound pulses and/or receiving ultrasound signals, the system may for example be configured to perform measurements of ultrasound properties of a sample by using the sub-array as a unit cell for sending and/or receiving ultrasound pulses and/or receiving ultrasound signals. In particular, ultrasound signals are detected as reflections of a previously generated ultrasound pulse, and imaging or other data processing is performed by mapping reflective properties of specific areas or locations.

The transducer array elements of a sub-array work as a combined functional unit, collecting 1D depth data sets by controlling the sending of an ultrasound pulse and collecting reflected ultrasound signals from a sample, e.g., from inside a patient's body. Collecting the data with this functional unit would be similar to a A-mode operation in ultrasound tools.

Also, the system may be configured to generate 3D spatial information from 1D data sets collected by the transducer array, or one or more sub-arrays of the transducer array.

The system may be configured to determine a position for a sub-array within the transducer array such that it is not hindered by impediments for ultrasound beams, e.g., due to ribs of a patient close to the surface, presence of lung tissue along the ultrasound transmission path or due to air bubbles between the transducer array elements of the sub-array and a sample. To collect data in order to generate, e.g., 3D spatial information, the control unit may be configured to control, for each sub-array, the location of the sub-array, as well as the focus depth and/or a steering angle for the sub-array depending on a chosen region of interest, e.g., within a patient's body.

For controlling a sub-array of transducer array elements in order to perform imaging, and, e.g., for measuring 3D spatial information, a positioning of the sub-array position is determined and controlled within the plurality of transducer array elements. This may be carried out by controlling a signal path between a switching array (e.g. a TFT array), which is arranged to transmit control signals to the transducer array elements and/or to transmit reading signals from the transducer array elements, and the control unit of the system, in particular a mixed signal ASIC, which may be arranged on the periphery of the sensor. The control unit, in particular the switching array, and a respective driver module for the switching array, may be configured to selectively switch rows and/or columns of transducer array elements to define an active sub-array and a sub-array location in a larger matrix of the system's transducer array.

In order to control direction and depth of a sub-array's ultrasound wave beam and controlling the ultrasound beam's profile, focusing or beam-steering or beam-forming module may be used. The beam profile is controlled by applying delays on the individual transducer array elements or by applying delays on a set of combined transducer array elements which provide a common input and output i.e. a functional unit. This may be performed both for sending ultrasound pulses, thereby focusing ultrasound energy at a depth defined by a predetermined focal point and sending the ultrasound energy under a predetermined steering angle. Analog to the sending, the beam profile may be controlled for receiving ultrasound signals, e.g., in order to define a direction and a focal depth for receiving ultrasound signals, e.g., from a predetermined location within a sample.

The complexity of the system may be defined as a characteristic in different ways. In particular, the complexity measures the number of individually driven functional units. For example, the "complexity" of the system may refer to the total amount of channels, which are used for generating ultrasound pulses and/or for receiving ultrasound signals.

The sub-array size is chosen and configured such that a suitable signal-to-noise ratio, a specific beam profile and/or a required image quality can be achieved. For example, when a certain sub-array size SA, where SA is the product of the number of rows "n" and columns "m" (SA = n*m), is chosen, this may reduce the complexity of the system significantly.

In an embodiment, the system comprises at least two transducer arrays forming a least one transducer array set; and the control unit is configured to control the transducer array set as one single transducer array. By using a transducer array set for example several positions can be monitored (e.g. needed for lung monitoring) by means of one system. Further, a greater surface area can be monitored. Also, different viewpoints for monitoring a target, which can be an organ, fluid carrying structure or other volumetric feature in the body e.g. the heart and/or lung and/or chest, can be established.

The transducer array units may be combined in series and/or in parallel into a single element, i.e., a single transducer array set, which is driven as a single transducer array of the ultrasound system.

In particular, the size and pitch of the transducer array units may be adjusted to the wavelength, which is used for the ultrasound, i.e., the sound wave length (lambda). Herein, the pitch denotes the spacing from the center of one transducer array element to the neighboring transducer array element. The ultrasound waves, as they are emitted from (or received by) these transducer array elements interfere differently for different pitch values. For a pitch above half of the wavelength that is used for ultrasound, the waves constructively interfere at angles outside of the "main lobe", therefore causing so-called "grating lobes". Thus, a pitch value of lambda/2 may be used in order to avoid grating lobes, which may negatively impact image quality when beam steering is carried out. However, a pitch value equal to lambda may also be used, if limited beam steering capabilities and some image ghosting effects are taken into account or corrected.

Different architectures may be used to address transducer array elements individually or combined in functional units. In a row column based architecture, for example, transducer array elements that are arranged in one dimension are combined and treated as a functional unit. Such elements in one dimension may be arranged along an essentially straight line. For example, instead of controlling the single elements of a transducer array individually and/or collecting signals from all transducer array elements, all elements along one axis may be combined into a combined input and/or output. For example, all elements of one row or column are combined. Connecting a transducer element to a TFT element configured as a switch, allows to selectively connect a transducer element to the row or column, so that a sub-selection can be made of the active transducers along a single row or column. The pulsers and receivers for driving respectively reading the combined transducers array elements can each be connected to either the rows or columns of the array or a combination thereof; leading to possible configurations in the read-out electronics which we designate as row/row, column/column or row/column read-out schemes.

The basic concept definition for an array with the number N rows and the number M columns (if N=M, then the array is a squared array, otherwise a rectangular array) is may be such, that there is a controller with a TFT element (the TFT element will open or close the signal path) and this controller defines, which rows are open or have to be open. Further, the controller defines or is aware of the columns, which are active for signal transmission and/or receiving, and the intersection of active N and active M is defining a sub-array. This way, the addressing of the transducer array elements can be done in a less complex way, as they will be addressed in the groups of transducer array elements which are called a sub-array.

In particular, the control unit may provide beam characteristics for each sub-array. Thus, the elements of this sub-array are used to generate ultrasound pulses in one specific direction and with defined pulse properties, respectively. On the other hand, the beam characteristics define ultrasound signal detection properties of the system, in particular the direction and depth where ultrasound signals are detected.

In an embodiment, a beam forming architecture is implemented by a hardware beam-forming module. In other words, the beam forming on a sub-array level is performed not or not only *via* signals from the control unit, but by a hard-wired chip design.

In particular, delays are defined for transducer array elements within one sub-array, such that a defined beam-form is reached for ultrasound signals generated or detected by these elements. In the analog domain, sensor signals for all sub-array elements are delayed and summed into a single output channel. Signals from this common output channel may be provided to one ADC and converted to a digital signal for further data treatment, filtering, imaging and monitoring of measurements.

The number of analog-digital converters that are needed by the system is reduced from one per receive channel to one per sub-array, thus also reducing the amount of data that is generated. Consequently, significant benefits in power consumption and data rate may be reached.

Additionally or alternatively, a software or programming implementation of sub-array beam-forming may be used in the system.

In an embodiment, the deformation calibration step comprises generating, and in particular sending, an ultrasound pulse by at least one transducer array element, and receiving an ultrasound signal by the same and/or another transducer array element. The received ultrasound signal may be a reflected signal by the generated ultrasound pulse. The deformation correction step also comprises determining the deformation information based on the generated ultrasound pulse and the received ultrasound signal.

Thus, the system itself provides advantageously the necessary information to perform a correction in order to take the deformation into account.

The generated ultrasound pulse may comprise a predetermined signal form, e.g., with a predetermined form, intensity, shape and/or dynamic form of a generated ultrasound emission. The ultrasound pulse may be generated by one or several transducer array elements, or the whole transducer array. Also, the ultrasound pulse may be generated by a sub-array, wherein a plurality of transducer array elements may be controlled as one single subunit of the transducer array in a coordinated fashion.

In particular, the received ultrasound signal may be detected such that it comprises a reflected signal of the previously generated ultrasound pulse. Also, the ultrasound signal may be received with a predetermined form, e.g., by adjusting the receiving characteristics of the transducer array element and/or applying a filter characteristic for limiting the received data to the predetermined characteristics. The ultrasound signal may be received by one or several transducer array elements, or the whole transducer array. Also, the ultrasound pulse may be received by a sub-array, wherein a plurality of transducer array elements may be controlled as one single subunit of the transducer array in a coordinated fashion.

When a flexible transducer array is applied by a force, such as e.g. from an adhesive, to a free-formed or curved surface, such as the body of a person, the array will tend to conform to the surface. This conformation will cause the patch to deform from its reference shape such that the deformed array will tend to follow the free-formed surface as close as possible. The reference shape of the transducer could e.g. be flat. When removing the forces which conform the transducer array to the free-formed surface, the array will tend to recover to its reference shape. The deformation of the array, when it's conformed to a surface, can in general not be known upfront, in particular when the array is applied to a body, as the shape of different people are in general not the same. The deformation of the array can cause unwanted effects in the ultrasound signals and the image information derived from them. These effects can be a geometrical distortion of the image and/or a blurring effect akin to defocusing. The function of the deformation correction module in the system is to correct for these deformation induced distortions.

The deformation correction module functions by analyzing the characteristics of the received ultrasound signal or the image data derived from these ultrasound signals, and deriving so-called deformation-correction information based on the analysis of these signals. The deformation correction information is the information needed for the algorithms to correct the distortion in the ultrasound signals and/or data due to the deformation of the transducer array. The process of correcting the image is also called the deformation correction step. The deformation correction information may either be derived directly from the ultrasound signals and/or images or alternatively the deformation correction module may first find an approximation for the "true shape" under which the patch is deformed, the true shape being the physical shape under which the patch is deformed after conforming it to a free-formed surface. The approximated shape, determined by the deformation correction module, is what we refer to as "deformation information". Based on this deformation information, the deformation correction information may then be derived and used to correct the ultrasound signal and or image data.

Following are specific embodiments of the methodology described in the previous paragraph.

a) An image metric may be derived from the ultrasound images, either globally or locally. The image metric is calculated for an assumed deformed shape of the ultrasound array. When the assumed shape is close or equal to the true shape an optimum is found in the image metric. The algorithms in the deformation correction module use this optimum to traverse a set of assumed deformed shapes, which we term the "shape-space" to find the approximation to the true shape (i.e. the deformation information). Once the deformation information is known the deformation correction step can be performed. The image standard deviation, its contrast or other sharpness metrics could be potential embodiments of such image metrics.

b) A correction may be performed without determining the actual shape of the transducer array nor directly correcting its estimate (so-called performing a shape-independent image correction for the transducer array). This can be used as a general means for correcting image data that is acquired by the transducer array, wherein the actual shape is not necessarily known. The image artifacts caused by the probe deformation are intrinsically dependent on the mismatch between the assumed deformation information and the true shape of the probe. Hence, unique reconstruction artifacts/errors are associated with different (wrong) deformation information. Therefore, filter with specific weights may be determined and/or other correction parameters may be determined for the transducer array and/or individual transducer array elements. By directly filtering the image data acquired with an unknown probe shape, for instance by taking the average of image data reconstructed assuming randomize probe shapes, one can directly estimate the true image data without directly recovering the probe deformation information.

c) Based on the method in b, the assumed shapes within the shape space are used to determine values for the deformation correction information. The deformation correction information is used to correct the ultrasound data and/or images, without actually establishing the deformation information related to the true shape, the corrected images are close to what would be obtained when the true shape would be known. The corrected data can be used to compare the data produced by an assumed shape in the shape space to the corrected data. When the assumed shape is close or equal to the true shape the comparison metric will be optimal. The comparison metric can be used to traverse the space shape in a tree-search based algorithm to find the approximation for the "true shape", i.e. the deformation information.

d) A sectorial image registration can be performed to determine the deformation information. For this procedure several sector image scans are performed from different positions on the transducer array, ideally combinations of two or more of these sector scans overlap. A sectorial image scan is a measurement where several pulse-echo measurements are performed from the same sub-array position, but each time with a different angle under which the ultrasound signal is send & received. The signals and/or images from overlapping sub-array positions can be used to spatially align the sub-arrays with respect to each other and as such find the deformation information. E.g. the different sub-array positions can aligned with respect to each-other in an image registration step, where features in the different sector scans images are compared to each-other. Furthermore, a principal component analysis of the curved surface of interest may be performed to find an simplified mathematical description of the possible surfaces shapes and as such simplify the search space for the registration algorithm, making it easier to find the correct alignment of the sector scans with respect to each other.

e) A time of flight measurement can be performed between two separate locations in the transducer array. Each of these locations could e.g. be measured by means of a separate sub-array, a so-called send- and receive subarray. For an undeformed, e.g. flat, ultrasound array sending an ultrasound pulse from the send-subarray to a known feature in the body would generate a signal in the receive-subarray when that receive-subarray is configured to receive from a specific location inside the body, i.e. the "receive direction". For a deformed subarray the receive direction will deviate from the receive direction for an undeformed array, and this deviation carries in it information on the deformation of the transducer array. Combining several of such send-receive measurements allow to reconstruct the deformation information and subsequently to perform the deformation correction step.

Once the deformation information is available to correct for the probe shape, the ultrasound signals arising from the individual transducing elements can be compensated for and the image data reconstructed. This primarily involves a spatial re-orientation of the ultrasound line data in 3D, after which existing ultrasound image reconstruction algorithms and/or compounding methods can be applied in order to generate a single 3D image of the imaged organ.

In the specific case of the embodiment b described above, the 3D image of the imaged organ can be directly estimated from a set of images reconstructed using assumed (wrong) deformation information. The mean image from this set of images, Im, is calculated by averaging the reconstructed image data, whereas the average pairwise difference, Id, is calculated as the pixelwise absolute difference between all the elements within the set of images reconstructed using assumed (wrong) deformation information. The final the 3D image of the imaged organ (Ir) can be then be estimated as Ir=Im-Id.

In an embodiment, a neural network, machine learning and/or artificial intelligence method is used to provide the deformation information based on provided sample imaging data with artifacts from a deformation of the transducer array.

In an embodiment, the deformation correction step further comprises providing the received ultrasound signal to a filter module and applying a low- and/or high-pass-filter, and determining at least one filter coefficient depending on the positional information of the transducer array element. This provides advantageously a more accurate and faster treatment.

The filter coefficient may comprise a weight parameter for a specific filter. In particular, a plurality of filters may be present and weights are determined to reach an improved filtering, deformation correction and/or calibration.

In particular, the deformation information comprises weight information and the filter module is configured to apply the weight information for applying different filters to the provided ultrasound signal.

For example, a plurality of measurements, i.e., sending ultrasound pulses and receiving the reflected ultrasound signals, is carried out and the filter coefficients, in particular weights or a plurality of weights, are determined and/or improved in an iterative approach.

The filter module may be configured to apply a combination of low- and high-pass filters, in i.e., based on randomly chosen imaging samples. Herein, the filter module may utilize weight parameters for the filter parameters and set these uniformly.

Improving on this method, a correlation between the randomly chosen samples may be determined and filter coefficients may be adapted for a more accurate filtering. By using a more accurate filter, filter coefficients and/or weights may generate more accurate solutions and/or it may help the computational method converge faster.

In an embodiment, the deformation correction step further comprises generating an ultrasound pulse, performing a beamsteering control for receiving a reflected of the ultrasound pulse under at least two angles and generating angle-dependent reflection information, and determining the deformation information based on the angle-dependent reflection information.

For example, when an ultrasound pulse is reflected from a diffusely scattering object or surface, such diffuse scatterer may be used as a reference point between a transmitting transducer array element and a receiving transducer array element, which are geometrically separated from each other. In particular, subarrays of the transducer array may be used instead of single transducer array elements. For example, the diffuse scatterer is located inside a sample volume such as a patient's body, at a first angle and/or distance with respect to the transmitting transducer array element. A deformation between the transmitting and receiving transducer array elements may be evaluated based on this angle.

Assuming a flat transducer array, the angle between the transmitting transducer array element and the scattering object determines a theoretical maximum receive angle under which a maximum readout signal may be received by the receiving transducer array element. Using beamsteering, the receiving transducer array element may be controlled to receive signals from a range of different angles, and the actual maximum receive signal may be determined. When the transducer array is not flat, the maximum will be shifted relative to the theoretical maximum receive angle. Applying this shift, a deformed position and/or orientation of the receive transducer array element is determined, and the shape of the deformed transducer array may be calculated.

In an embodiment, the deformation correction step further comprises performing a delay-based calibration and/or a time-of-flight calibration. This approach utilizes advantageously a difference between an expected delay, e.g., based on an assumed flat configuration of the transducer array and an actual, measured delay.

For example, a flat shape of the transducer array is assumed. When an ultrasound pulse is generated and sent towards a reflective surface, there is an expected delay between the signal sent by the transmitter transducer array element or subarray, and the time of receiving the reflected ultrasound signal by the receiver transducer array element or subarray.

If the transducer array has a curved shape, by analyzing the difference between a measured delay for an actual transducer array with a curved shape and a theoretical delay for a flat transducer array, the curvature of the transducer array is estimated.

In particular, the intensity of the received signals may be improved by using a Hadamard coding to deconstruct the reflected signals and avoid a "beaming" effect, when the wave reflects off a surface, such as the surface of a scattering object within a sample volume.

For example, a method for time-of-flight calibration can comprise some or all of the following steps, in this or another order:

a) A distance between a transmitting transducer sub-array and a scatterer is measured by transmitting and receiving an ultrasound signal at the same sub-array location. Beam-forming may be used to tilt a transmitted ultrasound beam (Tx) by a certain angle (-x degrees) and to tilt a received ultrasound beam (Rx) by another certain angle (x degrees).

Subsequently, an L1 minimization problem is solved to determine the biggest scatterer's position, and the distance is calculated.

b) A distance between a receiving transducer sub-array and the scatterer is measured by transmitting and receiving another ultrasound signal at different sub-array locations. To this end, the transmitted ultrasound beam (Tx) is tilted by a certain angle (-x degrees), and a sectorial scan is done on the receiving ultrasound beam (Rx) such that it points to several directions.

For each direction, the L1 minimization problem is solved to determine the biggest scatterer's position, and the distance is calculated.

This distance is calculated for a plurality of directions, and outlier measurements are discarded.

c) Based on the distances of steps a) and b), a relative position is determined for the receiving (Rx) sub-array by trigonometry.

In another embodiment, principal component analysis (PCA) may be used to perform sectorial image registration for ultrasound self-shape estimation and image compounding.

The method may comprise some or all of the following steps, in this or another order:

a) A parametric model is provided, representing the shape of the ultrasound transducer array. Optionally, prior knowledge about an acceptable physiological range of shape variations is used in the parametric model.

b) Also, an ultrasound scanning sequence is provided that uses sub-apertures from the whole transducer array to acquire images.

c) An image registration algorithm is executed. Local metrics of agreement are used to assess, whether the different images are properly aligned.

Such a method allows advantageously using the parametric shape model to constrain the image registration algorithm. Thus, the alignment of the sub-aperture images is in agreement with a given physiological range of the anatomical surface to which the probe is adhered to. For example, the surface of a subject's skin or chest cannot be assumed to be deformed beyond a physiologically relevant range.

Moreover, the method advantageously reduces the degrees of freedom for solving the overall problem, thereby contributing to its efficient and robust implementation.

For the current estimate of the transducer array shape, the sub-aperture images are distributed spatially considering the estimated location and orientation of the individual transducer elements. A metric of local agreement is then calculated for all the image positions, therefore quantifying if the content of all sub-aperture images is locally similar or not. In an embodiment, a standard deviation of the samples of all the aligned images within a local region may be used as a metric for local agreement.

The shape of the transducer array may advantageously be updated iteratively. An optimization of the local agreement metric or the entire shape space can be exhaustively sought for the real array shape.

Also, the invention relates to a method for operating an ultrasound system. Herein, a deformation correction step is performed, wherein deformation information is determined for the transducer array.

In particular, the method serves the purpose of operating the ultrasound system. Thus, it has the same advantages as the ultrasound system of the invention.

The invention will be better understood with the aid of the description of an embodiment given by way of example and illustrated by the figures.

It is shown in

**Fig. 1**      an embodiment of the ultrasound system;

**Fig. 2A-D**   detailed views of the transducer array of the embodiment of the ultrasound system;

**Fig. 3A-D**   another embodiment of the ultrasound system;

**Fig. 4**      another embodiment of the ultrasound system;

**Fig. 5**      an embodiment of a method to operate the ultrasound system;

**Fig. 6**      another embodiment of a method to operate the ultrasound system;

**Fig. 7**      another embodiment of a method to operate the ultrasound system;

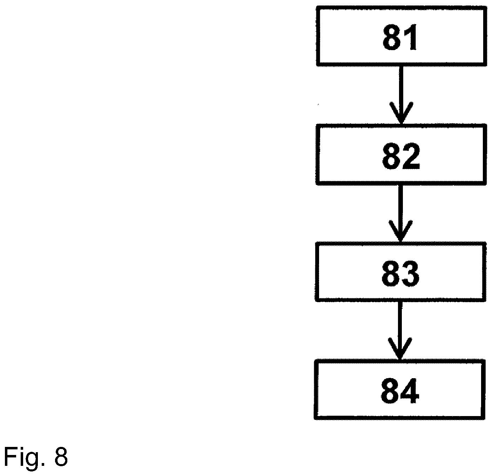

**Fig. 8**      an embodiment of a method for correction of ultrasound systems; and

**Fig. 9A-K**   embodiments of methods for calibration of the ultrasound system.

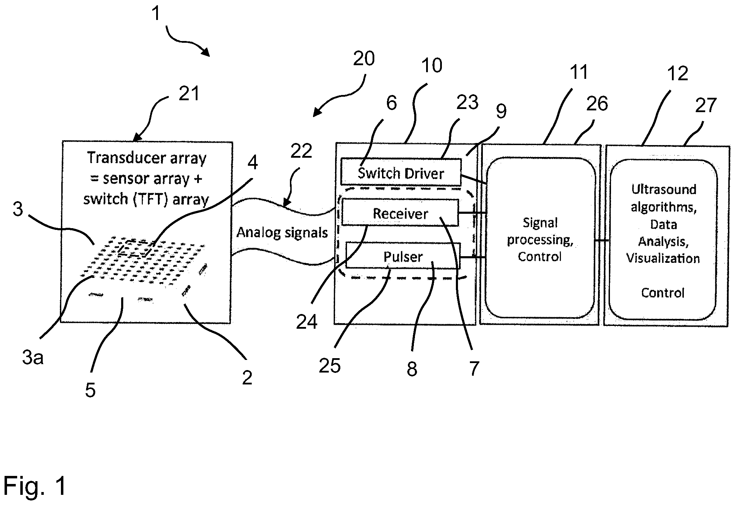

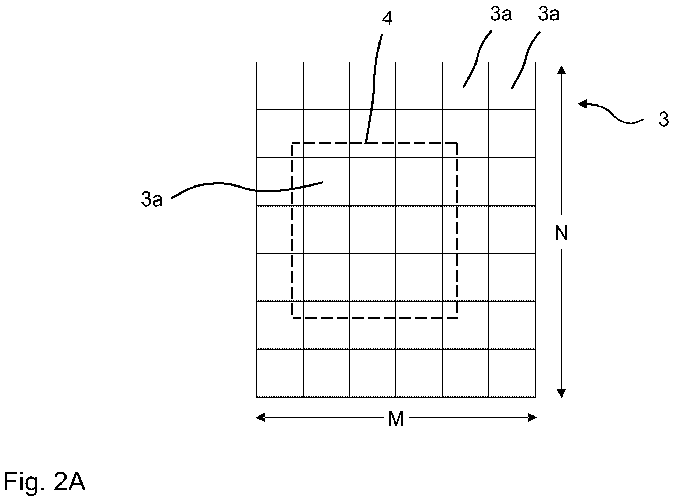

Turning to **Fig. 1** and **Fig. 2A** to **Fig. 2C,** an embodiment of the ultrasound system is described.

**Fig. 1** shows a schematical overview and explaining the functional blocks of the ultrasound system 1.

The ultrasound system 1 according to the embodiment is configured as a wearable ultrasound system 1, in particular for long-term monitoring of a patient's cardiac activity.

In particular, the ultrasound system can be in general an ultrasound system as disclosed by WO 2019/162485 A1. The below additional features can be further realized in this already disclosed ultrasound system of WO 2019/162485 A1.

The ultrasound sensor and support electronics can be integrated in a flexible body worn format, as it is shown in this embodiment.

In the embodiment as shown in **Fig. 1,** the ultrasound system is (at least partially) carried in a patch 2.

The ultrasound system 1 comprises a transducer array 3, which is configured as a piezoelectric micromachined ultrasonic transducer (pMUT) array 3 in the present embodiment.

The transducer array 3 comprises a plurality of transducer array elements 3a.

In the present embodiment pMUT elements 3a.

A subset of the transducer array elements 3a forms a sub-array 4.

In particular, each transducer array element 3a can consist of one or several sub-elements 3b, in particular pMUT sub-elements 3b or components, which are driven as a single larger transducer array "element" 3a.

In other embodiments, capacitive micro-machined ultrasonic transducer (cMUT) components can be used.

Other configurations with different numbers of transducer sub-elements 3b or a different arrangement of the sub-elements 3b are possible as well.

Furthermore, there is a switch array or switch module 5, which can more specific be embodied as a TFT array.

A further part of the ultrasound system 1 is the Analog Front-End 10 with the Switch Driver 6, the Receiver 7 and the Pulser 8.

The Switch Driver 6 can be embodied as a TFT driver module.

The Receiver 7 can be embodied as analog-digital converter (ADC).

The Pulser 8 can be a pulser module.

There can be a digital application-specific integrated circuit (ASIC) 9.

Further, there is an interface module 11 and an external unit 12/back-end unit 12.

A transducer array 21, which comprises a sensor array (e.g., like the transducer array 3 described above) and a switch/TFT array, is coupled with a unit implementing a switch driver 23, a receiver 24 and a pulser unit 25.

These units are configured to communicate via analog signals 22 with the transducer array 21.

In the embodiment, they are implemented in application-specific integrated circuits. Such ASICs can be located on the periphery of the sensor or on the Analog Front-End 10.

Also, they are used to receive and treat the signals, and communicate with a signal processing/control unit 26, which is implemented in an embedded compute system, e.g. on the Analog Front-End 10 with CPU, FPGA with memory or other units, these control and signal processing algorithms might also be implemented partly or completely in an ASIC.

Further processing of provided data is performed by a control module 27, incorporating for example ultrasound image reconstruction algorithms, data analysis, visualization and control algorithms.

In the case of a system 20 wired to an external unit 12, such as a PC, at least a part of the treatment algorithms can optionally be run on the embedded compute system and/or on the external unit 12. In a wireless case, such computational steps can be carried out in the embedded compute system or potentially at least partly in a holder.

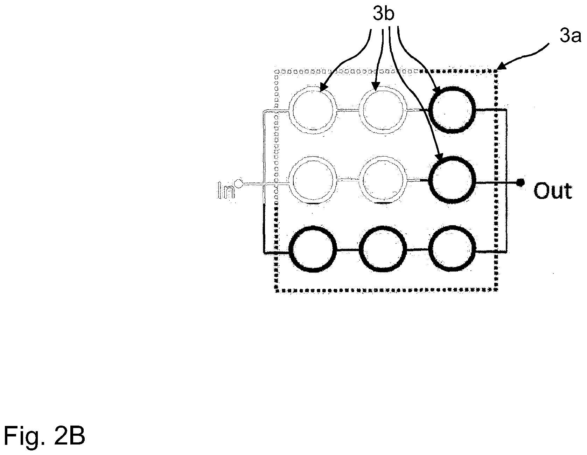

In **Fig. 2B,** one example of a possible transducer array element design is shown: Nine sub-elements 3a (pMUT, cMUT or other elements) are configured to form one transducer array element 3a. To this end, they are arranged in three rows and three columns. Therein, the sub-elements 3 of one row are connected in series, and the rows of sub-elements 3a in parallel. Thus, three parallel branches of each three sub-elements 3b connected in series are connected. Other configurations are possible as well. In total, the transducer array element 3a has one "In" and one "Out" line. Herein, the sub-elements 3b are not controlled individually, but the transducer array element 3a is controlled as a whole.

In the present embodiment, the transducer array 3 comprises 150 * 150 pMUT elements 3a, which are arranged in a 2D matrix with perpendicular rows and columns. The array size may differ in different embodiments.

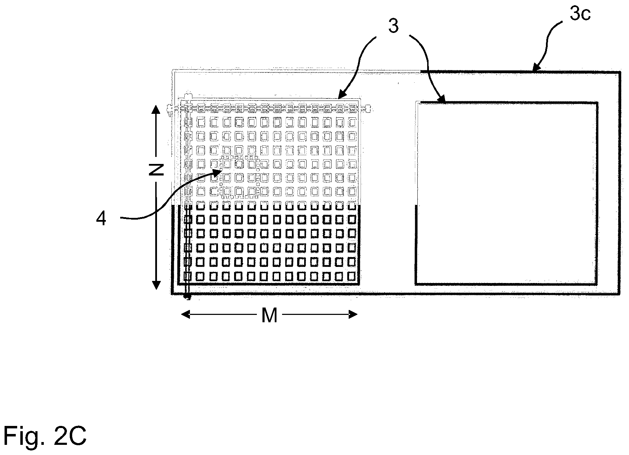

Furthermore, **Fig. 2C** shows another embodiment, wherein two or more transducer arrays 3 can form a transducer array set 3c, which is then controlled as one functional unit, i.e. "virtual" single transducer array.

In the embodiment of **Fig. 2C,** a first and a second transducer array 3 are comprised by one transducer array set 3c. In this embodiment, the transducer array set 3c is controlled as "virtually" one single transducer array 3.

Also, **Fig. 2C** shows that the transducer array 3 comprises a plurality of transducer array elements 3a, which are arranged in a matrix with a number of N rows and M columns.

Furthermore, **Fig. 2C** shows that a subset of the transducer array elements 3a make up a sub-array 4, which is herein controlled as one unit. The sub-array 4 has a number of n rows and m columns.

**Fig. 2C** also shows that elements 3a along a row and/or a column of the transducer array 3 are interconnected with one or more interconnecting lines, a so called row/column based architecture. The elements 3a connect in parallel to the interconnects, with a switch, in particular a TFT switch, determining if an element 3a is connected or not and to which signal path it connects. Thus, rows and/or columns of the transducer array 3 can be controlled together. This allows simplifying the system by switching rows and/or columns of elements 3a together.

The transducer array 3 of the embodiment is flexible. Here, the transducer array elements 3a are arranged on a flexible substrate, e.g., comprising a flexible polymer material.

Furthermore, the ultrasound system 1 comprises a switch array which can be embodied as a thin-film transistor (TFT) array 5, comprising a plurality of TFT elements.

The switch array 5 is arranged such that its switch elements (or TFT elements when embodied as a TFT array) control corresponding pMUT elements 3a. In particular, each TFT element is assigned to one pMUT element 3a.

In the present embodiment, a TFT array 5 with 150 * 150 TFT elements is used, which are arranged in a 2D matrix with perpendicular rows and columns. The array size depends in particular on the size of the respective pMUT array 3, and it may differ in different embodiments.

Similar to the transducer array 3, the TFT array 5 of the embodiment is also flexible and/or stretchable. Here, the TFT array elements are arranged on a flexible substrate, e.g., comprising a flexible polymer material.

In particular, the TFT array 5 is arranged on the same substrate as the transducer array 3.

In particular, the integration may be monolithic or heterogeneous. In the present exemplary embodiment, the sensor layer and TFT layer may be fabricated separately and joined in a wafer-to-wafer bonding process, followed by the processing of an interconnect module.

In particular, "Heterogeneous Integration (HI)" may refer to the assembly and packaging of multiple separately manufactured components onto a single chip in order to improve functionality and enhance operating characteristics. In the present embodiment, one single array is created with a single functionality (sensing), implying a monolithic device.

In particular, attention is drawn to **Fig. 2C,** wherein an example of a row/column architecture is demonstrated.

In the embodiment, both the pMUT array 3 and the TFT array 5 are integrated into an adhesive patch 2.

In the embodiment, the adhesive patch 2 has a size of about 10x10 cm. The size may vary in different embodiments.

The adhesive patch 2 comprises a material that is suitable for the purpose of attaching the ultrasound system 1 and in particular the transducer array 3 to a patient's skin. Therefore, biocompatible and breathable materials are used. The adhesive layer on the patch is used to attach the patch to the body and to make sure that the position of the transducer array 3 is essentially constant with respect to the patient's skin.

In another embodiment, an "acoustic matching layer" is provided in between the skin and the transducer array 3.

The system 1 also comprises a functional block for the analog control of the electronics, a so called Analog Front-End.

The Analog Front-End 10 is coupled with the transducer array 3 and the TFT array 5 for providing options such as control and read-out for the transducer array 3 and/or the TFT array 5. To this end, both data and electrical power may be transferred over the coupling between the Analog Front-End 10 and the patch 2 with transducer array 3 and TFT array 5.

According to the embodiment, the Analog Front-End can be implemented in an application specific IC (ASIC) or as circuits on the TFT layer or as discrete components or a combination thereof. All the physical components of which the analog front-end is comprised can be either integrated directly on the TFT as integrated components, on the periphery of the TFT as IC's or on a PCB (flex or rigid) physically attached to the sensor 2 through physical interconnects.

In different embodiments, the application specific IC's may be either integrated on the periphery of the TFT layer or on the PCB.

Here, a TFT driver module 6 is provided on the Analog Front End 10, which is configured to provide a low-level interface to control and manage the TFT array 5 and its individual TFT elements, respectively. In an embodiment, a gate driver is integrated with the ASIC on the periphery of the TFT layer. In another embodiment the driver circuitry could be designed partly or completely in the TFT layer.

In particular, the TFT driver module 6 of the present embodiment can be integrated together with send and receive modules for controlling the generation of ultrasound pulses and detecting ultrasound signals, respectively. All these functional modules form part of the analog front-end.

Also, a receiver 7 is provided as part of the analog-front end 10, which is configured to convert an analog input signal to a digital output signal.

Also, a pulser module 8 is provided as part of the analog front end 10, which is configured to control the transducer array 3 such that ultrasound pulses are generated.

In particular, a beam-forming is performed here. Delay values may be set individually for each pulser unit of the system 1 that generates an ultrasound pulse. The delays on the different channels, in particular each pMUT element 3a, of the pulser unit will determine the focusing and the beam profile.

Also, a digital middle-end is provided , which is configured to perform part of the control operations for the ultrasound system 1, and specifically to control the activity of the transducer array 3 and the TFT array 5 for generating ultrasound pulses and detecting ultrasound signals. The digital middle-end is also configured to perform signal processing on the signals received from the analog front-end.

As part of the digital middle end, microprocessors may be provided and/or other units for performing operations for controlling the ultrasound system 1 and its electronic components.

Thus, the ultrasound system 1 of the embodiment provides an easily customized ultrasound sensor and monitoring device.

The interface module provides the possibility to build up a connection based on a wire connection and/or a wireless connection. In the present embodiment, examples for connection methods include USB and Ethernet as well as via Bluetooth and WiFi networks.

The interface module is here configured to allow for data communication and/or power connections with external units.

In the embodiment, the PCB with its components and the TFT array 5 constitute an integrated control unit and data processing unit. PCB and TFT array 5, and the transducer array 3, may in different embodiments, be configured as units that are formed separately or they may be incorporated in a common unit.

In a further embodiment, a remote or cloud based back-end unit 12 may be accessed through the interface module, either directly or indirectly via another device, such as a computer that is connected to a local or wide area network. The back-end unit may perform operations such as to reading and storing data, data analysis, configuration of the device, providing further information and administrating access the acquired data based on an authentication system.

Turning to **Fig. 2A,** a detailed view of the transducer array 3 of the embodiment of the ultrasound system 1 is described.

The transducer array 3 has a matrix of pMUT elements 3a, which are only schematically shown in a small number.

The system 1 is configured such that a sub-array 4 of pMUT elements 3a may be defined. The elements 3a of a sub-array 4 are treated as one functional unit for generating ultrasound pulses and/or detecting ultrasound signals.

In the embodiment, an ASIC performs steps for defining sub-arrays 4 within the transducer array 3. The definition of sub-arrays 4 may be carried out in different ways, e.g., by way of the chip design or by other units controlling the transducer array 3.

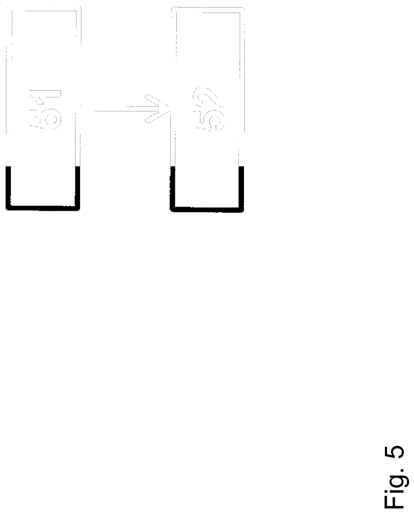

In the present embodiment, the transducer array 3 has N=150 rows and M=150 columns, which are ordered in a two-dimensional matrix. On the other hand, a sub-array size value of 16 rows by 16 columns is defined. Thus, groups of 162 elements 3a are grouped into one sub-array 4. This leads to a considerable reduction in system complexity and data volume, respectively. This aspect is further described below with reference to **Fig. 5.**

Herein, the interrelated elements 3a are chose such that they are neighboring to each other.

In further embodiments, this definition of sub-arrays 4 may be configured in different ways, such as, in a configuration step, by inputting a predetermined sub-array size value, which defines the number of pMUT elements 3a to be included in a sub-array 4, and/or by inputting a location of a sub-array 4 within the pMUT array 3. Also, the form of a sub-array 4, i.e., its dimensions size, may be determined by an input value.

In one embodiment, the sub-array size may be defined during the system development. In the products itself, the sub-array size can be fixed, without an option to dynamically reconfigure this value; in this case, a fixed size would be used.

Said values to control how sub-arrays 4 are defined may also be determined by the digital Middle End and/or digital back-end, part of this functionality might be implemented in an ASIC or another control element of the ultrasound system 1, or by an input over the interface module, in particular depending on an algorithm. Such an algorithm for determining parameters for defining the sub-arrays 4 may use input values such as data/image quality requirements, a signal-to-noise ratio, information about factors interfering with the operation of the system, user input and configuration information, and/or other parameters.

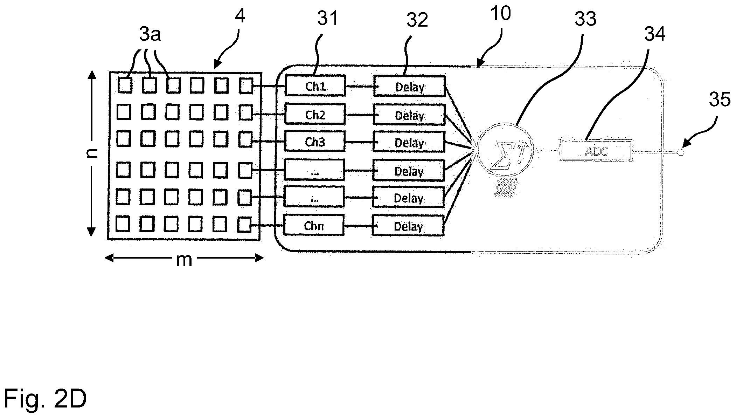

Turning to **Fig. 2D,** an embodiment for implementing a row/column based micro beam-former is schematically described.

A sub-array 4 has a number of n row and m columns, and is connected to a micro beam-former 30. In this specific case, the n rows of transducer array elements 3a are combined into one output channel 31 each. These output channels are named "Ch1" to "Chn".

For the transducer array element 3a of the sub-array 4, the micro beam-former 30 combines the n analog input channels 31 from the sub-array 4 into one single digital output channel 35.

To this end, a delay operation is performed by delay units 32 and a sum unit 32 is subsequently used to coherently add the n analog inputs together.

An analog-digital converter 34 is used to convert the resulting analog signal to a digital signal, which is output through a digital output channel 35.

In further embodiments, each channel 31 can do some analog processing of the input signals before its signal is combined in the analog beam-former 30.

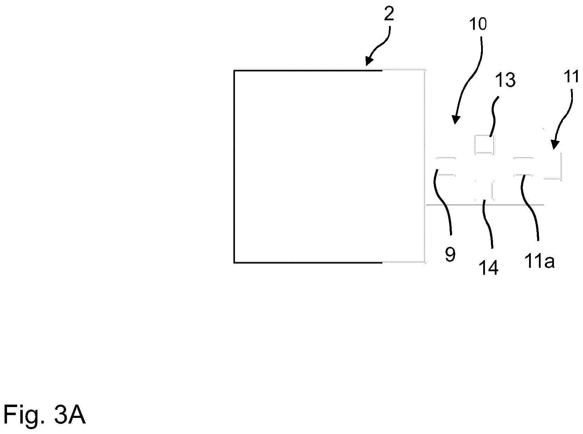

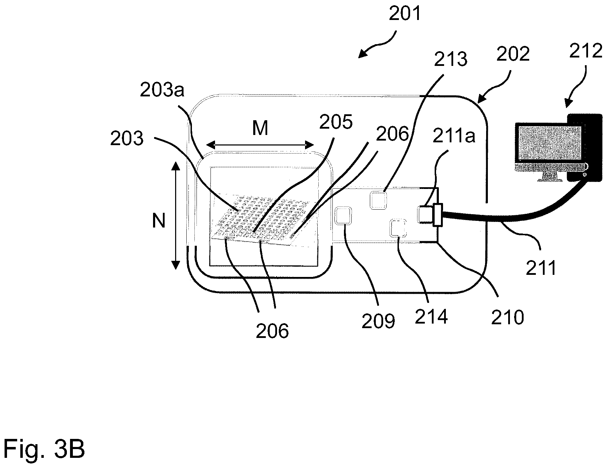

Turning to **Fig. 3A** to **Fig. 3E,** other embodiments of the ultrasound system are described. Reference is made to the embodiments of the ultrasound system as described above. Figures 1 and 3A to 3D are meant to present analog embodiments of the system, although different aspects are highlighted and different elements are explicitly shown.

In **Fig. 3A,** a PCB 10 is shown connected to the patch 2. The transducer array 3 and the TFT array 5 are not shown, since they are also integrated with the patch 2. Also, the interface module 11 is directly connected to the PCB 10.

On the PCB 10, circuits for controlling the system 1 are shown.

The ASIC 9 is provided on the PCB 10.

Also, an interface control module 11a is provided and implemented with another IC. It provides a low-level interface to the interface module 11, thereby allowing to control the interface module 11.

Also, a memory module 13 is provided, herein specifically configured as a random access memory (RAM) module 13.

Also, a power management module 14 is provided.

In another embodiment, a power source is provided, for example a battery as further explained below with reference to **Fig. 3E.** In this case, access to the stored power is provided to the system components via the power management module 14. In this case, the interface module 11 may be configured to provide a wireless data connection, in particular to a control unit.

In the present embodiment, another way of providing power to the system may be used, such as a connection to an external power source or an external power grid.

The ASIC 9, interface module ASIC 11a, the memory module 13 and the power management module 14 may be implemented in different ways, some of which may be known in the art. For example, a microprocessor, an ASIC chip and/or other units may be used.

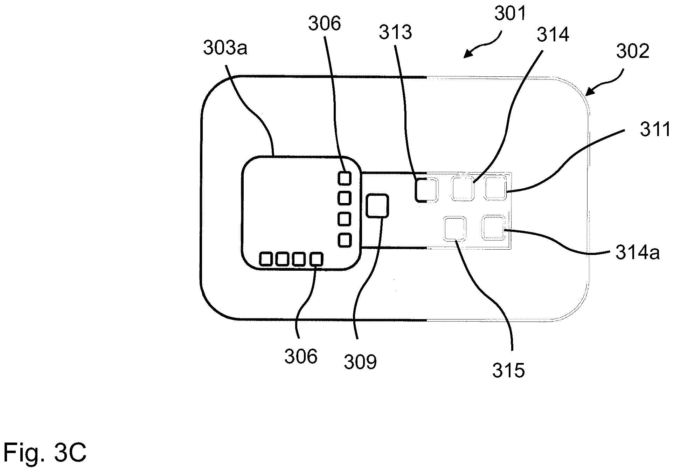

Turning to **Fig. 3C,** another view of an embodiment equivalent to the one of **Fig. 3A** is described.

In this embodiment, a system 201 comprises a unit 203a, in which a transducer array 203 and a TFT array 205 are integrated. A flexible PCB 210 is coupled to this unit 203a, and both are integrated in a patch case 202.

The PCB 210 might be rigid, flexible, stretchable or a hybrid combination thereof.

The system 201 contains a module 206 with an ASIC with 16 channels, containing an analog front-end functional block and optionally a complete or partial digital middle/back end. The ASIC module 206 can be integrated either on the sensor unit 203a or on the PCB 210.

In particular, the flexible PCB 210 is configured such that it is flexible on the top of the sensor, in particular on top of the sensor unit 203a. The physical link between the PCB 210 and the sensor unit 203a being made by interconnects such as in particular flexible interconnects.

The flexible PCB 210 comprises components for an embedded computer system, such as a control device 209, comprising for example CPU, FPGA and/or other elements.

The flexible PCB 210 further comprises a physical interface module such as e.g. a USB connector 211 and a USB transceiver 211a, by which the system 201 is coupled to a PC 212.

The flexible PCB 210 also comprises a RAM module 213 and a power management IC 214.

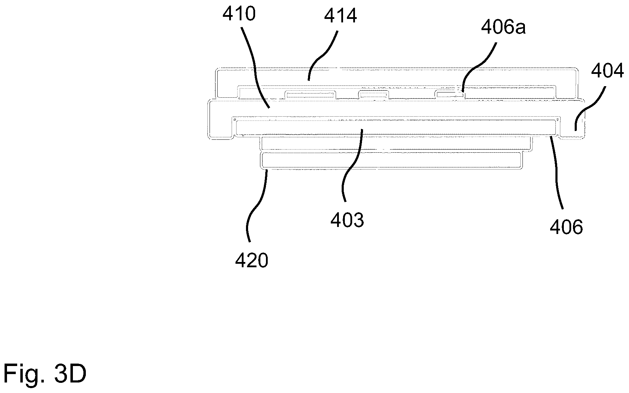

Turning to **Fig. 3D,** another view of an embodiment equivalent to the one of **Fig. 3A** is described.

A system 301 comprises a flexible packaging, in particular a patch case 302, into which a sensor unit 303a and a flexible PCB 310 are integrated. The flexible PCB 310 is coupled to the sensor unit 303a.

The PCB 310 might be rigid, flexible, stretchable or a hybrid combination thereof.

The system 301 contains a module 306 with an ASIC with 16 inputs, containing an analog front-end functional block and optionally a complete or partial digital middle/back end. The ASIC module 306 can be integrated either on the sensor unit 303a or on the PCB 310.

The flexible PCB 310 has a control device 309, a wireless transceiver 311, a memory module 313, a battery gauge 314, a wireless/wired charger 314a and a power IC 315 integrated on it.

In particular, the flexible PCB 310 is configured such that it is flexible on the top of the sensor, in particular on top of the sensor unit 303a. The physical link between the PCB 310 and the sensor unit 303a being made by interconnects such as in particular flexible interconnects.

Also, a flexible battery may be provided.

Turning to **Fig. 3E,** a schematic cross-section of an embodiment, such as the embodiment of **Fig. 3D,** is described.

The system 401 comprises a sensor 403.

An acoustic matching and/or glue layer 420 is applied on one side onto the sensor 403, to enable good contact to a subject's skin.

On the other side of the sensor 403, a periphery 406 section is arranged. In particular, electronics for controlling the sensor 403 are arranged in the periphery 406.

As a layer above the periphery 406, interconnects 404 are arranged. In particular, the interconnects 404 can connect elements of the sensor 403, e.g., in a row/column architecture.

In a layer above the interconnects 404, IC elements 406a are arranged.

On top of the above-described arrangement, a flexible battery 414 is arranged. This flexible battery 414 might also be arranged next to IC elements 406a and/or the PCB 410.

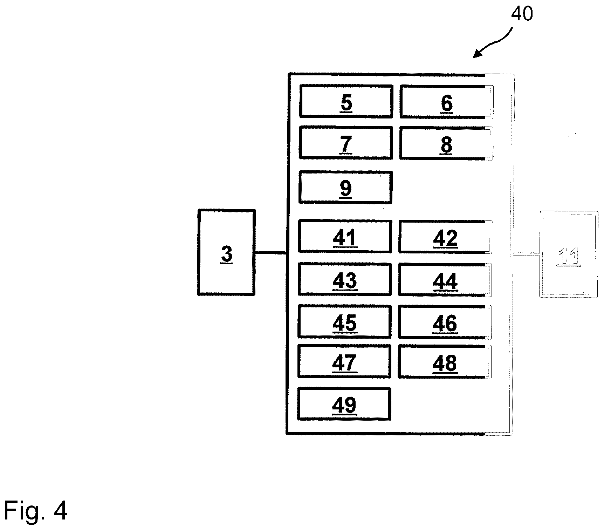

Turning to **Fig. 4,** a schematic view of another embodiment is shown. The shown components may also be present in the embodiments described above; similar or equivalent elements have the same reference numerals and are not described again in detail.

Some or all of the elements and modules shown in **Fig. 4** may be implemented in different units, such as separately formed devices, or they may be implemented by way of program modules within the same computational unit and/or a digital memory device.

The combination of elements and modules of this embodiment is to be understood as exemplary, and as comprising a large number of optional features, which are not necessarily linked to each other or to be combined in one embodiment of the invention.

In **Fig. 4,** the transducer array 3 is shown, which is linked to a control unit 40 (controlling the transducer array 3), which may be configured at least partially on a PCB 10.

The connection between the transducer array 3 and the control unit 40 is a direct and cable-bound connection. However, in an alternative embodiment, an indirect and/or wireless connection is possible.

For instance, the control unit 40 can be attached to the patch 2 (possible for wireless patches and wired patches). Further, there can be an additional control unit in a separate computer, in particular for the wired version.

The transducer array 3, configured for providing ultrasound waves and/or receiving ultrasound signals, comprises a plurality of transducer array elements 3a.

The control unit 40 comprises the TFT array 5, which was already described above.

The control unit 40 further comprises a dedicated data processing unit 41, which may in different embodiments comprise some of the other modules (as specified below with reference numerals 41 to 49) or which may in different embodiments be implemented by several individual modules. The data processing unit 41 receives and processes data from the transducer array 3.

Moreover, the control unit 40 comprises a reference module 45.

Also, the control unit 40 comprises a volume reconstruction module 46, in particular for reconstructing the volume of a target organ or part of a target organ which is in this embodiment the left ventricle of a subject equipped with the ultrasound system 1.

Furthermore, the control unit 40 comprises a selected monitoring unit 47, in particular for monitoring the left ventricle.

Further, the control unit 40 comprises a correction module 44.

Also, the control unit 40 comprises a complexity reduction module 42.

Additionally, the control unit 40 comprises a deformation correction module 43.

In addition to that, the control unit 40 comprises an artifact determination module 48.

Furthermore, the control unit 40 comprises a phase determination module 49.

The system 1 according to the embodiment is controlled by integrated support electronics. The support electronics comprise mixed signal ASIC elements distributed along the periphery of the sensor. The sensor may comprise the transducer array 3. The ASIC elements could also be located on the PCB 10.

Also, the rest of the electronics is integrated on the flexible PCB 10, which is in turn connected to the sensor.

The ASIC elements are distributed along the edge of the flexible ultrasound sensor and have some or all of the following functions:

- Actuating the mechanical transducers, i.e., the transducer array elements 3a;

- Processing and digitizing signals received by the transducer array elements 3a;

- Controlling the switching of the TFT array 5; and/or

- Dynamically reconnecting the analog channels, i.e., from different transducer array elements 3a, to different sets of columns/rows.

The flexible PCB 10 contains a digital "master" ASIC 9 and/or a microprocessor, for performing digital signal processing, and control of the other ASIC elements. The digital signal processing and/or control may also be implemented in part or completely on the mixed signal ASICs distributed along the periphery and/or edge of the sensor (e.g. routed to the backside of the sensor on a PCB by means of interconnects).

By performing row/column addressing of the pMUT elements 3a of the transducer array 3, the complexity of the system 1 is reduced from N*M to N+M, wherein N and M represents the amount of rows and columns in the array. In case of a fully wired array (i.e., when each transducer array element 3a is controlled individually) each element 3a can be addressed individually (hence the N*M complexity), while for a row/column based architecture the elements 3a along the same row or column are connected together and addressed as a single unit, reducing the complexity to N+M. Each unit could still consist of multiple interconnect lines forming the connections between the external nodes and the internal array elements. Each interconnect line can carry a different signal, e.g. to provide a signal line, a ground line or to provide a control signal for switching the TFT transistors.

A hardware beam-former may be used to combine, e.g., 16 analog channels, which connects each to an interconnected unit of transducer array elements 3a, to a single ADC 7, i.e., one digital channel. The number of combined elements 3a determines the factor by which the system complexity is reduced. In particular, a plurality of ADCs 7 may be provided and these may be linked to a defined sub-array 4 of transducer array elements 3a.

In the embodiment, a sub-array 4 is used as one functional unit, e.g., a "single entity" for scanning the full array (a unit cell). This single entity is electronically controlled and/or scanned over the larger transducer array 3. In particular, the sub-arrays 4 are electronically "shifted" per single row/column of the transducer array 3.

In the present embodiment, a sub-array 4 is electronically controlled as a single unit. In particular, there is one ASIC for each sub-array 4.

In further embodiments, multiple sub-arrays 4 may be provided for each ASIC. The number is determined by the accessible power, cost restrictions, acceptable compactness and/or speed requirements for the system.

By using several ASICs for controlling individual sub-arrays or controlling multiple sub-arrays per ASIC, faster scanning is possible, since several measurements, in particular "single entities" or "functional units", are operated in parallel.

In the following, aspects and methods of operating an embodiment as described above are described. In particular, the following description is based on an embodiment similar to the one of **Fig. 4**.

When the transducer array 3 is placed on the body (thorax) of the subject, it does not have any prior information on the features of the body on which it is placed. Thus, the reference module 45 provides a detailed scanning of the region of the total transducer array 3 (matrix of transducers). This scanning will allow to detect the left ventricle as feature of interest/target. Any method known in the art can be used for feature detection, e.g., correlation with known feature, neural networks etc. Furthermore, this scanning allows to detect incorrect placement of the transducer array 3 on the thorax of the subject, and/or it may be evaluated, whether the left ventricle is only partially visible from the position of the transducer array 3.

Based on the reference ultrasound data obtained by the reference module 45, the volume reconstruction module 46 reconstructs the volume of the left ventricle by providing a collection of collimated ultrasound beams by a sub-array 3a of the transducer array 3.

Thus, the volume reconstruction module 46 can control a location of a sub-array 4 within the plurality of transducer array elements 3a. Also, the volume reconstruction module 46 can control, for each sub-array 4, a focus depth and/or a steering angle for an ultrasound sending beam profile and/or an ultrasound receiving beam profile implemented by the sub-array 4 of transducer array elements 3a; such a beam-forming algorithm may be implemented as a software module for a programmable unit and/or by chip design, e.g., in an ASIC.

Another method could use different sparse pulse-echo measurements of the left ventricle, and use these measurements to determine the deformation against a more detailed reference volume, which would have been collected in an earlier measurement, i.e., it would track the deformation of the reference volume over time using only sparse measurements. Yet another method could use the imaging data directly to track the deformation directly against a reference image.

The artifact determination module 48 can be used for identification of artifacts and avoiding obstructions to ultrasound imaging caused by artifacts, such as air bubbles between the transducer array and the skin of the patient equipped with the system or bones, e.g. ribs. In particular, strong ultrasound reflection of the ribs may be used for identification. The artifact determination module 46 in general may combine groups of transducer array elements 3a into a sub-array 4.

Combining of groups of transducer elements 3a into a sub-array 4 can alternatively be done by the complexity reduction module 42.

Also, the complexity reduction module 42 may be implemented as a software module, comprising instructions for computational operations that implement the function of complexity reduction. Such a software module may in particular be implemented together with the control unit 40.

Specifically, the complexity reduction module 42 may be comprised, at least partially, by the control unit 40 and/or by the data processing unit 41.

Further, the selected monitoring unit 47 is configured to monitor the left ventricle (by means of the sub-array 4).

In particular, the computational load may be further reduced by only performing a detailed scanning of the volume at discrete time instances of the cardiac cycle:

The phase determination module 49 is configured for determining cardiac cycle phases in particular end-systolic phase and end-diastolic phase, as for evaluation of the stroke volume only the end-diastolic and end-systolic phases are of importance. For this a single (alternatively a few) ultrasound pulse-echo measurement(s) is/are done through a carefully chosen position of the left ventricle or other reference, from which the cardiac cycle can be determined. This is done several times through the cardiac cycle so that a good graph can be obtained of the contraction over time, e.g., 40 to 200 "samples" could be taken over the full cardiac cycle. From these sampled curves, the end-diastolic and end-systolic phases are detected by analyzing the derivative to determine min/max in the curve(s). Alternatively, several cycles can be measured, which allows for accurate estimation of the end-systolic/diastolic phases. The regularity of the heart rhythm then allows to determine the next instance of the end-diastolic/end-systolic phases. Another option may be to train a neural network, which takes as input the time series and gives as an output the end-diastolic and systolic phases. Yet another option would be to identify focus on a feature of the heart which provides a good indication of the heart cycle, such as e.g. a valve of a heart chamber. The phase can also be determined by monitoring the heart cycle with other signals like e.g. an ECG signal.

Therefore, a detailed volume scanning at the end-diastolic and end-systolic phase is enabled by means of the selected monitoring unit 47.

For instance, once the end-diastolic and systolic phases have been detected by the phase determination module 49, the system 1 can switch from the phase determination module 49, and send a single "scanning" beam to the volume reconstruction module 46 and send a sufficient amount of beams to reconstruct the volume.

For data treatment, in particular for monitoring and/or clinical applications, a cardiac output variation over certain time periods, e.g., over a day, may be determined by the system 1. Also, motion data may be provided and, e.g., linked to measured cardiac activity, such as heart rate and other parameters. For example, an accelerometer may be integrated or connected to the system 1. Also, a movement of the patient's thorax may be determined with an accelerometer. Furthermore, the influence of breathing and physical activity may be considered. A movement of the patient may be measured, e.g., a rhythm of breathing. Furthermore, the lungs may be monitored by the ultrasound system as well.

Also, changing properties of the transducer array 3, e.g., a pMUT array 3, may be analyzed in order to obtain information on the deformation of the transducer array 3.

Also, an automatic evaluation may be carried out to determine whether the signal-to-noise ratio is too low, i.e., below a predetermined limit, and the reliability of measurements is affected.

In particular, ultrasound based pulse-echo measurements using focused beams may be used to sample a probe volume.

As a way to reduce the volume of acquired data, which needs to be processed and analyzed during monitoring of cardiac activity, sampling may be limited to end-diastolic and/or end-systolic volumes of a heart chamber of or another volume.

In order to track a heart rhythm, a single reference beam may be used to track heart motion and evaluate end-diastolic and/or systolic phases.

To make sure that the monitoring provides most reliable data, different measures may be taken: Ultrasound pulse-echo measurements may be used together with a sampling of selected, reduced measurements of few spatial data points. Also, ultrasound pulse-echo measurements may be used together with a varying pulse repetition frequency, wherein the pulse repetition frequency is reduced outside of defined measurement windows. The pulse repetition frequency may relate to a regular interval; also, pulse-echo measurements may be limited to well-defined time windows. For example, a higher pulse repetition frequency may be set within these time windows, in order to gather more pulse-echo measurements and perform a denser sampling of the volume on interest, while the pulse repetition frequency is set lower outside the time windows, e.g., to track the phase of the heart contraction with lower time resolution.

The monitoring may comprise, in particular with a given frequency, steps of calculating a volume, calculating the volume of ellipse in order to approximate a volume and/or volume change, segmentation and integration of image data, detecting a base line value, calibrating the system and/or monitoring strain, in particular on the transducer array, in order to estimate deformation and/or a dynamic form factor of the transducer array.

In particular, monitoring and calibration are implemented as two distinct steps in the operation of the system, in particular where the transducer array is included in a flexible patch:

a) The patch is applied to a patient's body.

b) The patch would perform a calibration step (detecting its deformed shape due to the shape of the thorax).

c) The system would perform monitoring and tracking the volume of the heart over time.

During monitoring, potentially one would need to correct for dynamic shape changes of the patch, e.g., due to breathing or movement of the patient. This could be done by, e.g., a) performing a recalibration at regular intervals, b) measuring deformation with strain monitoring, c) using a tracking algorithm for the shape deformation.

The monitoring of the volume could be done in different manners:

a) Establish a detailed baseline shape of the ventricle, and track the changes in this baseline shape over time with limited number of ultrasound based pulse-echo measurements.

b) Measure with a limited number of ultrasound based pulse-echo measurements and establish directly the volume from these.

In particular, image based metrics may be used for the calibration step. This may enable a deformation-free reconstruction of images even from a deformed transducer array, i.e., without reconstruction of the concrete form factor of the transducer array. E.g., wrong shapes may be assumed and true images may be reconstructed based on these wrong shapes.

Also, shape estimation for the transducer array may be performed based on deformation-free reconstruction data.

Measurements may be carried out by generating reference beams towards a diffuse reflector, and detecting the reflected ultrasound signals with another known sub-array 4, in order to evaluate displacement of the sub-arrays 4 relatively to each other, which would allow for estimating the deformed shape of the array 3.

Also, changing properties of the transducer array 3, e.g., a pMUT array 3, may be analyzed in order to obtain information on the deformation of the transducer array 3.

Different detection methods may be used, in particular for monitoring cardiac activity in a patient: A left ventricle may be detected and targeted for measuring, in particular measuring its volume and the dynamic change in volume.

Moreover, ribs and other obstructions in the patient's body may be detected in order to reduce noise in the measurements and to optimize the choice of a suitable ultrasound beam-forming and focusing. In particular, strong ultrasound reflection of the ribs may be used for identification.

In order to define a sub-array 4 of the transducer array 3, several steps may be provided and carried out, individually or in combination with each other. A positioning step may be carried out for sub-arrays 4 in order to control their position within the transducer array 3, i.e., for choosing suitable elements 3a and assigning them to the sub-array 4. Also, a sub-array's position may be shifted, in particular randomly, to avoid long term stress on specific elements 3a, leading to degradation of the elements, in particular pMUT elements 3a.

The use of ASIC elements may improve the security of the device, since the respective control methods are not easily manipulated, at least not without direct physical access to the device.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a complexity reduction module 42, which is configured to define at least one sub-array 4 of transducer array elements 3a and to control the at least one sub-array 4 as a functional unit for sending at least one ultrasound pulse and/or receiving at least one ultrasound signal.

In one embodiment, the ultrasound system 1, in particular for cardiac monitoring, comprises a transducer array 3 with a plurality of transducer array elements 3a for providing ultrasound waves, a control unit 40, which is coupled to the transducer array 3, for controlling the transducer array 3, and a data processing unit 41 for receiving data from the transducer array 3 and processing the received data. Therein, the system further comprises at least one reference module 45 configured for obtaining reference ultrasound data, at least one volume reconstruction module 46 configured for reconstructing the volume of a target organ or part of a target organ, and at least one selected monitoring unit 47 configured for monitoring the volume of the target organ and/or part of the target organ.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 has at least two operating modes, namely a sleep mode and a measuring mode. The control unit 40 is configured, when the sleep mode is activated, to have a low frequency system clock running at a predetermined sleep frequency, and, when the measuring mode is activated, to set a high frequency system clock to a measuring frequency.

In one embodiment, the ultrasound system 1, in particular for cardiac monitoring, comprises a transducer array 3 with a plurality of transducer array elements 3a, wherein the transducer array 3 is flexible, a control unit 40, which is coupled to the transducer array 3, for controlling the transducer array 3, and a data processing unit 41 for receiving data from the transducer array 3 and processing the received data. Therein, the system 1 further comprises at least one correction module 44 configured for correction of effects in analog ultrasound signals caused by bending of the flexible transducer array 3.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a deformation correction module 43, which is configured to perform a deformation correction step, wherein deformation information is determined for the transducer array 3.

The embodiments may be freely combined with each other. Further modules as described above, for example with reference to figure 4, may be provided optionally to allow further functionalities as described in this specification.

Turning to **Fig. 5,** a method for operating the ultrasound system 1 is described. The following description is based on the embodiment of the ultrasound system 1 described above with reference to, e.g., **Fig. 4,** but other embodiments of the ultrasound system 1 may be suited as well.

In this context, "complexity reduction" denotes a reduction of the amount of read and/or write channels needed to address the sensors, in particular the transducer array elements 3a or a sub-array 4.

This can be reached by

a) combining sub-elements 3b into a single functional element 3a (cf. **Fig. 2B**);

b) connecting elements 3a together in a row/column architecture, leading to a complexity reduction from N*M to (N+M) (cf. **Fig. 2C);**

c) driving a sub-array 4 instead of the full transducer array 3 at once, leading to a complexity reduction from (N+M) to (n+m); and/or

d) using a micro beam-former to combine the n (or m) analog input channels 31 of a sub-array 4 into a single digital input channel 35 (cf. **Fig. 2D**).

In a step 51, a plurality of sub-arrays 4 is defined within the transducer array 3. In a subsequent step 52, the individual sub-arrays 4 are controlled as functional units for sending ultrasound pulses and/or receiving ultrasound signals.

In the system of the embodiment, the steps 51, 52 are performed by the complexity reduction module 42, which is comprised by the control unit 40 of the ultrasound system 1. Herein, the complexity reduction module 42 is configured to control other units and modules, especially the TFT driver module 6, the receiver module 7, and/or the pulser module 8, depending on whether a send or a receive cycle is active.

The steps 51, 52 may be performed in a cyclic manner, i.e., they may be repeated for example at given intervals or triggered by predefined events and/or control signals.

In one embodiment, the system is configured such that the sub-array 4 is defined dynamically and may change its position.

In the present embodiment, the sub-arrays 4 are defined such that there is no overlap between the transducer array elements 3a of separate sub-arrays 4, i.e., no transducer array elements 3a are "shared" among different sub-arrays 4.

In another embodiment, a transducers array set 3c comprises at least two transducer arrays 3 and the control unit 40 is configured to control the transducer arrays 3 as one functional unit.

Also, in an embodiment, the transducer array 3 may be controlled according to a row/column based architecture.

Furthermore, a sub-array size may be predetermined or provided or determined by an algorithm, and sub-arrays 4 of the sub-array size are defined based on this value. The transducer array 3 is then operated based on these individual sub-arrays 4.

Also, the transducer elements 3a are controlled by the control unit 40, in particular via the TFT array 5. The transducer array elements 3a of each sub-array 4 detect an analog ultrasound sensor signal, and produce an analog signal. This analog signal is provided to the receiver module 7.

In particular, analog signals from the transducer array elements 3b of one sub-array 4 are processed by one common receiver module of the system 1. In further embodiments, different receiver modules 7 may be provided, e.g., in order to speed up data acquisition and processing.

Furthermore, a micro beam-forming architecture can be implemented by a hardware beam-forming module 43, such as a specialized ASIC element and/or another element of the control unit of the system 1.

Also, data may be filtered after it has been received from the transducer array 3. This may be carried out by a filter module 44, which may act on analog and/or digital signals. Depending on which filter is applied, filtered data is obtained with a reduced spatial and/or temporal resolution.

In another embodiment, a filter can be configured by adjusting filter properties. Thus, filters may be activated and deactivated, the impact of a filter on the data may be adjusted and/or a resolution of the filtered data may be adapted, e.g., to the needs for monitoring specific organs and functions, imaging purposes, or device-specific properties.

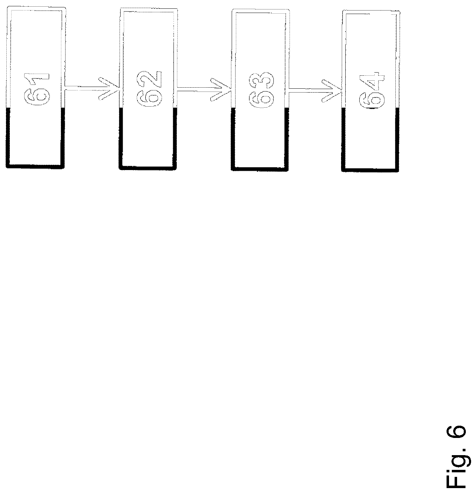

Turning to **Fig. 6,** another embodiment of a method for operating the ultrasound system is described. The following description is based on the embodiment of the ultrasound system described above with reference to, e.g., **Fig. 4,** but other embodiments of the ultrasound system 1 may be suited as well.

In a first step 61 of the method, a measuring mode of the ultrasound system is active. Thus, the system is configured to perform measurements using the transducer array 3.

In particular, the measurements are controlled by a system clock, which is comprised by the control unit 40. This system clock provides a frequency at which measurement steps such as generating ultrasound pulses and receiving ultrasound signals, as well as processing and analyzing of collected data are carried out.

In a step 62, the control unit 40 receives a sleep signal and subsequently activates a sleep mode of the system. According to the embodiment, the sleep signal is generated after a predetermined time duration, wherein no measurements were carried out by the system and/or wherein no data queries were received. In another embodiment, the sleep signal is received from an external unit via the interface module 11.

In response to the sleep signal, a sleep-on sequence is performed, and during this sequence, the control unit 40 shuts down a series of modules, except those that are necessary to power up the system in a later step. In particular, a wake-up controller module, an interrupt controller module, and/or a timer module are kept running during the sleep mode.

With the sleep mode activated, the system clock is configured to set a standby frequency to a value between 25 and 50 kHz, preferably to a value of 32 kHz.

In another step 63, a wake-up signal is received and the control unit 40 is configured to activate the measuring mode of the system. To this end, a sleep-off sequence is performed, wherein modules for performing measurements and providing data are subsequently started. In particular, the sleep-off sequence may be configured such that those modules, which were deactivated during the sleep-on sequence, are activated again.

According to the embodiment, the first module to be activated comprises a phase-locked loop (PLL), which is configured to provide a measurement frequency signal to the system clock. Thus, the system will advantageously be provided with a high frequency clock after performing the sleep-off sequence.

With the measuring mode activated, the system clock is configured to set a measurement frequency to a value of about 200 MHz, preferably between 150 and 250 MHz.

In a further step 64, the system 1 is configured to perform periodic measurements for monitoring the cardiac activity of a patient. These measurements are timed by the system clock, which is at a higher frequency value and allows high-frequency measurements.

In one embodiment, a common system clock is used to provide the low or high frequency in sleep and measuring mode, respectively. Thus, when the measuring mode is activated, the system clock is set to the measuring frequency, and when the sleep mode is activated, the system clock is set to the sleep frequency.

In another embodiment, a low frequency system clock and a high frequency system clock are provided. Therein, the low frequency system clock has the sleep frequency, while the high frequency system clock is set to the higher measuring frequency, once the measuring mode is activated.

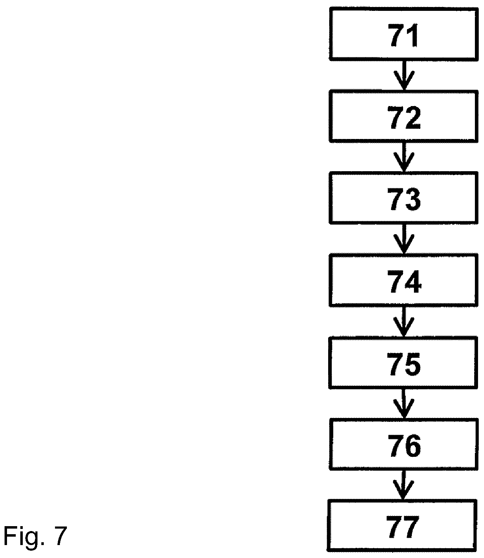

Turning to **Fig. 7,** another embodiment of a method for operating the above-described ultrasound system 1 is described. The following description is at least in principal based on the embodiment of the ultrasound system 1 described above with reference to, e.g., **Fig. 4,** but other embodiments of the ultrasound system 1 may be suited as well.

The method comprises the steps 71-77.

In the system 1 of the embodiment, steps 72 and 73 are obtained by the reference module 45, step 74 is obtained by the artifact determination module 48, step 75 is obtained by the phase determination module 49, step 76 is obtained by the volume reconstruction module 46 and step 77 is obtained by the selected monitoring unit 47.

The respective modules/units are comprised by the control unit 40 of the ultrasound system 1.

The steps 72, 73, 74, 75 76 and/ or 77 may be performed synchronously. Further, steps 72-77 can be performed in a different order as mentioned below.

The steps 72-77 may be performed in a cyclic manner, i.e. they may be repeated for example at given intervals or triggered by predefined events and/or control signals.

In a step 71, a transducer array 3 with a plurality of transducer array elements 3a for providing ultrasound waves is placed on the body of a subject.

In a step 72, a reference ultrasound image is obtained by scanning.

In a step 73, a target organ and/or part of a target organ is detected (in this embodiment, the left ventricle).

In this embodiment, step 72 comprises two different phases of scanning which differ in terms of resolution. In particular, step 72 is characterized by starting with a faster scanning (low-resolution scanning), which is sufficient for detecting the general region of interest. A more detailed scanning of this region of interest follows, with a higher resolution, to more accurately detect e.g. features, artifacts and/or regions which need to be monitored (i.e. the region of the left ventricle). In general, there may be more than two different phases of scanning differing in terms of resolution. Also, a constant increase of resolution may be generally possible.

Based on the results of step 72, the left ventricle is detected in step 73.

In a step 74, possible artifacts such as rips and/or air bubbles between the transducer array 3 and the skin of the subject are identified. Obstructions to ultrasound signals caused by such artifacts may be avoided by reconfiguration of a sub-array 4, cf. **Fig. 4.**

In a step 75 cardiac cycle phases are determined, in particular the end-systolic and the end-diastolic phase.

In a step 76, the volume of the left ventricle is reconstructed.

In the embodiment, step 76 comprises scanning at least partially the volume of the left ventricle without producing an image.

The scanning may be based on ultrasound based pulsed echo measurements with focused beams, wherein ultrasound beams are beam-formed by selecting a subset of transducer array elements 3a of the transducer array 3 as a sub-array 4 within the transducer array elements 3a and applying different phases to individual and/or sets of transducer array elements 3a. Data related to a single pulse-echo measurement provides the information of two boundary points of the volume of the left ventricle, wherein the volume can be reconstructed from a point cloud based on interpolation between different points of the point cloud.

Also, the volume may be reconstructed using the sparse point cloud and determining the deformation against a more detailed reference volume which would have been collected in an earlier measurement, i.e., tracking the deformation of the reference volume over time with sparse measurements. Yet another method could use the imaging data directly to track the deformation directly against a reference image.

In a step 77, the volume of the left ventricle is monitored, only at the end-systolic and the end-diastolic phase.

Note that the target organ (here the left ventricle) may in general comprise a heart or blood vessel and the part of the target organ may comprise a ventricle or atrium. In addition to targeting an organ other volumetric features inside the body may be imaged, such as e.g. the volume of a blood inside the body due to e.g. internal hemorrhaging.

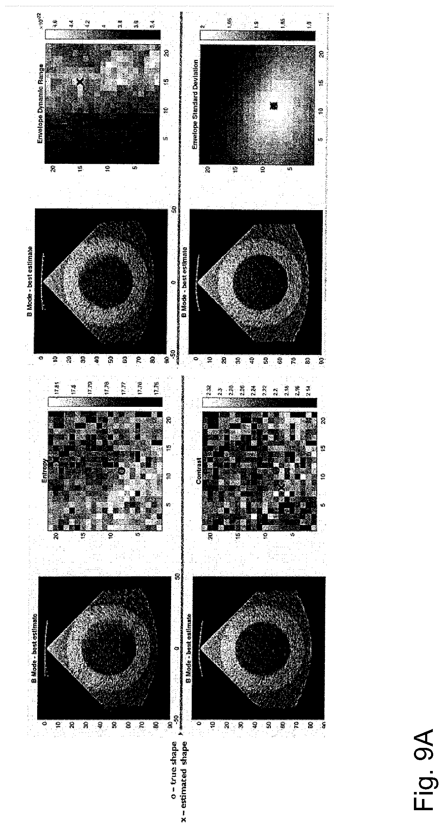

Turning to **Fig. 9A** to **Fig. 9C,** further embodiments of methods for operating the above-described ultrasound system are described. In particular, methods to perform a deformation correction step are described. The following description is based on the embodiment of the ultrasound system described above with reference to, e.g., **Fig. 4,** but other embodiments of the ultrasound system 1 may be suited as well.

In this system, the following deformation correction and/or calibration steps are carried out by a deformation correction module 43, as shown for example in Fig. 4. In further embodiments, this step may be carried out - at least partially - by other modules and elements of the system.

**Fig. 9A** relates to a first exemplary embodiment.

Autofocus metrics can be used for quantifying errors arising from image reconstruction, when a wrong shape of the transducer array 3 is assumed. These metrics can be used as surrogates for the error of the estimated probe shape. Hence, in the present embodiment, different metrics are used as optimization criterion in a shape estimation method for a flexible ultrasonic transducer array.

Autofocus, contrast and image sharpness metrics enable the use of iterative shape refinement schemes, by posing the self-shape estimation as an optimization problem, where the shape is updated in a gradient ascent/descent fashion. In the present embodiment, previously proposed autofocus metrics (minimization of B-mode image entropy and contrast maximization), as well as original ones (dynamic range and standard deviation of log-compressed envelope data) were assessed as optimization target functions.

For the example shown in **Fig. 9A,** three in-silico phantoms (cyst/point standard phantom, concentric ring phantom and a realistic simulated left ventricle), each having a different probe shape (128 elements, pitch=312nm, f0=2.5MHz) were simulated in Field II. In order to study the accuracy of the global optimum, overall smoothness and the existence of local optima, the aforementioned autofocus metrics were estimated in a 2D parametric shape space containing the true probe conformation. The 2D parametric shape space ranged from a flat surface to curved concave shapes, with the curvature of each half of the probe being controlled in a 2D grid.

Fig. 9A shows exemplary results from a comparison of recovered probe shapes (cross) versus true probe shape (circle), and the associated feature variation over the tested shape space. Herein, the mean root mean square error of the shape estimates associated with the global optima (after iterative closest point estimation for rigid alignment) were 0.48 $\pm$0.17 mm and 0.13$\pm$0.07 mm, for B-mode image entropy and contrast respectively, and 0.34$\pm$0.21 mm and 0.066 $\pm$0.058 mm for log-compressed envelope dynamic range and standard deviation. In particular, log-compressed envelope standard deviation presents the smoother variation over the entire parametric shape, which facilitates the convergence of an iterative solver to find the global optimum. Hence, it may be used as an autofocus metric enabling online self-shape estimation of conformal, flexible ultrasonic transducer arrays 3.

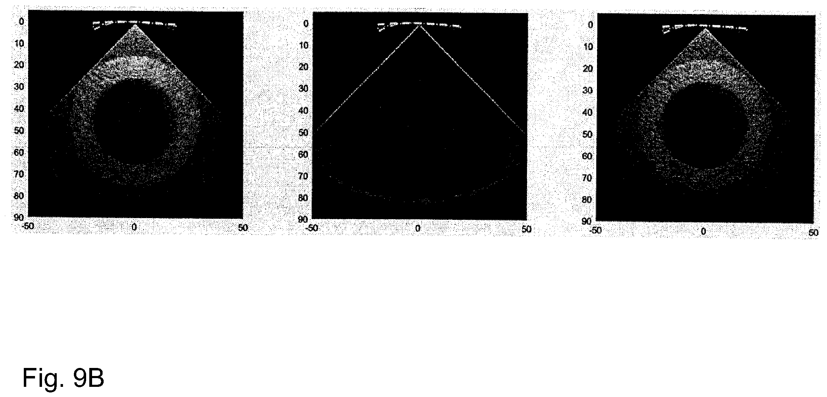

**Fig. 9B** relates to another exemplary embodiment.

Shape-independent image reconstruction may be performed for ultrasound imaging with a flexible transducer array 3.

This embodiment describes how to deal with a wrong assumed shape of the transducer array 3, and how to correct it and how to reconstruct a correct image from the acquired image data.

Conformal ultrasound imaging through flexible probes is increasingly gaining attention in the medical arena. However, image reconstruction in such setup requires precise information of elements position for accurate image reconstruction via conventional delay-and-sum beam-forming. However, this is far from trivial, either requiring external sensing strategies to decode the flexible probe shape, e.g., through fiber deformation sensors, or through image-based autofocus metrics for self-shape estimation of the ultrasonic transducer array. In the present embodiment, a simple reconstruction approach is applied, based on the combination of multiple images reconstructed with random estimated shapes, demonstrating that the resulting reconstruction is stable regardless the sampled wrong shapes used in the reconstruction process.

The proposed method is based on the observation that, when the assumed shape of the transducer array is wrong, the reconstructed image is blurred. However, since the reconstruction artifacts are intrinsically associated with the estimated probe shape, sampling a number of N wrong probe shapes yields N reconstructed images with different artifacts.

A mean image may be estimated from this set of images, Im, as well as the average pairwise difference, Id, from:

$$I_m(x,z) = \frac{1}{N}\sum_i I_i(x,z) \qquad (1)$$

$$I_d(x,z) = \frac{1}{M}\sum_{i\neq j}\left|I_i(x,z) - I_j(x,z)\right| \qquad (2)$$

where M equals the total number of combinations of N images, 2 images at the time. Since the mean image will be blurred due to the existence of image reconstruction artifacts around the true object boundaries in the image, subtracting Id from Im will suppress these artifacts, enabling the resulting reconstructed image to be closer to the true image.

Hence, the proposed reconstruction approach yields:

$$I_r(x,z) = I_r(x,z) - I_d(x,z) \qquad (3)$$

Three in-silico phantoms (cyst/point standard phantom, concentric ring phantom and a realistic simulated left ventricle), each having a different probe shape (128 elements, pitch=312nm, f0=2.5MHz) were simulated in Field II.

Without loss of generality, a 2D parametric shape space was considered containing the true probe conformation, from where 4 shapes of the transducer array were randomly sampled (one per quadrant) to estimate the true image. The 2D parametric shape space ranged from a flat surface to curved concave shapes, with the curvature of each half of the probe being controlled in a 2D grid. This process was repeated 100 times and the resulting images compared with the ground truth image generated with the true probe shape.

An illustrative example of the proposed concept is shown in **Fig.** 9B. Images are reconstructed with 4 random probe shapes, a mean image, Im, as well as a pairwise average difference, Id, are used to suppress reconstruction artifacts. The average correlation of the proposed reconstruction approach yield 0.466±0.073, 0.729±0.053 and 0.898 ±0.021 for the three test phantoms respectively. Similarly, no loss of contrast was observable across the regions of the reconstructed images with the proposed method, whereas lateral resolution was found to be similar as in the true image. Hence, it provides a promising route for conformal ultrasound imaging setups.

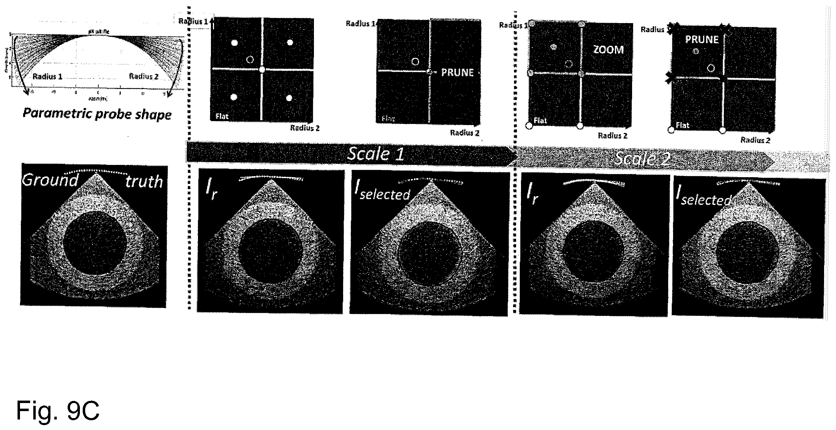

**Fig. 9C** relates to another exemplary embodiment.

Herein, a shape estimation of a flexible ultrasonic probe, i.e., a transducer array, is performed using intrinsic image reconstruction artifacts. In particular, a detailed approach is shown how to reconstruct the shape of the probe by employing image artifacts and applying a shape space pruning algorithm.

Conformal ultrasound imaging through flexible transducer arrays is increasingly gaining attention in the medical arena. However, conventional delay-and-sum image formation requires external sensing strategies to decode the flexible probe shape, e.g., through fiber deformation sensors, or alternatively image-based autofocus metrics for self-shape estimation of the ultrasonic transducer array, e.g., B-mode image entropy or contrast. Nonetheless, such metrics remain fairly dependent on the actual image content, having therefore an unpredictable behavior, whereas external sensing strategies add on to the system cost. In the present work we aim to avoid the pitfalls of such solutions and hence propose an internal autofocus metric, based on the intrinsic reconstruction artifacts, in order to identify the most likely probe shape.

The method of the present embodiment is based on the observation that, when a wrong shape of the transducer array is assumed, the reconstructed image is blurred. However, these errors can be minimized by estimating a reconstructed image, Ir, from the mean image from a set of reconstructed images by using different assumed shapes of the transducer array, Im, as well as the average pairwise difference, Id, as:

$$I_r(x,z) = I_r(x,z) - I_d(x,z)$$

$$I_m(x,z) = \frac{1}{N}\sum_i I_i(x,y)$$

$$I_d(x,z) = \frac{1}{M}\sum_{i\neq j}\left|I_i(x,y) - I_j(x,z)\right|$$

where M equals the total number of combinations of N images, 2 images at the time.

Since the mean image will be blurred due to the existence of image reconstruction artifacts around the true object boundaries in the image, subtracting Id from Im will suppress these artifacts, enabling the resulting reconstructed image to be closer to the true image. By computing the correlation of each of the N individual images with Ir, one can estimate which image has the most accurate image content, and hence the smallest error in the corresponding assumed probe shape.

$$R_i = corr(I_r, I_i), \rightarrow I_{selected} = argmin\{R_i\}$$

Without loss of generality, a 2D parametric shape space was considered containing the true probe conformation of the transducer array. The 2D parametric shape space ranges from a flat surface to curved concave shapes. A multiscale tree pruning was used to trim the 2D shape towards the most likely probe shape of the current set, based on iterative estimations of Ri. Three scales were using in the pruning process, each having 5 images to estimate Ir, as shown in **Fig. 9C.**

Three in-silico phantoms (cyst/point standard phantom, concentric ring phantom and a realistic simulated left ventricle), each having a different probe shape (128 elements, pitch=312 nm, f0=2.5 MHz) were simulated in Field II. The average root mean square error of the shape estimates associated with proposed method was $0.07 \pm 0.02$ mm for the tested phantoms, which represent approx. 2% of the maximum shape amplitude in the Z direction. Hence, a promising route is provided for self-shape estimation in conformal ultrasound imaging setups based on flexible transducer arrays.

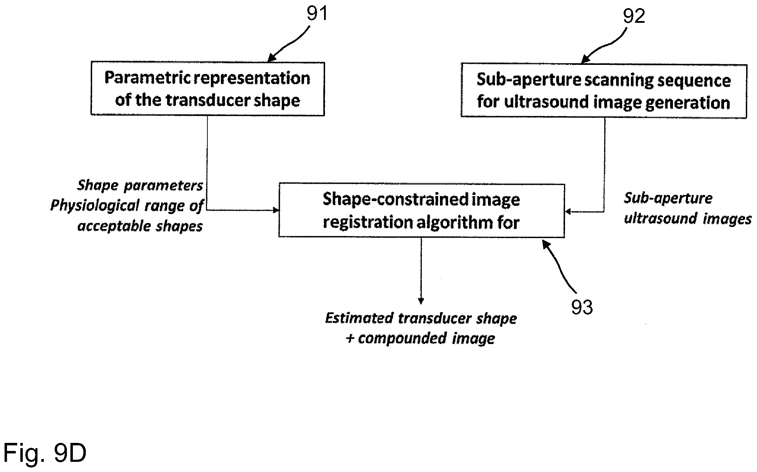

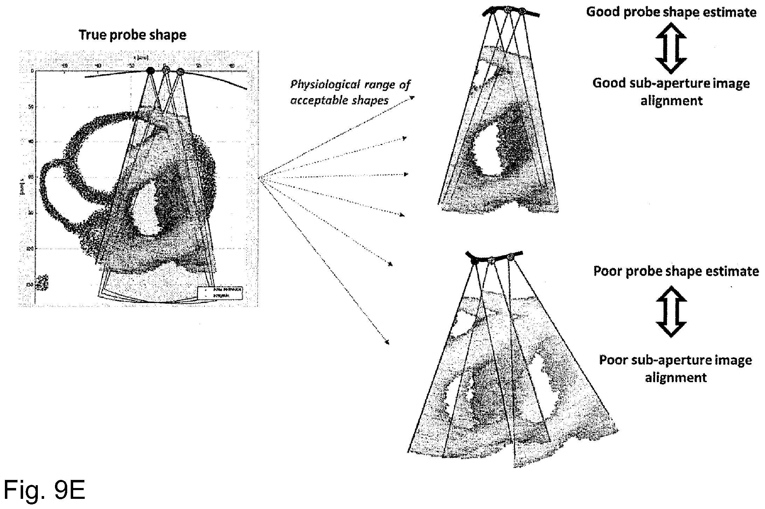

Turning to **Fig. 9D** and **Fig. 9E,** an embodiment of a method for principal component analysis (PCA)-driven sectorial image registration for ultrasound self-shape estimation and image compounding is described.

Reference is made to components of the system as described above, e.g., for **Fig. 1** and **Fig. 2A to Fig. 2D.** However, other system designs may be utilized as well.

The method comprises three main blocks parts, as shown in **Fig. 9D:**

1. In a step 91, a parametric model is provided to represent the shape of the ultrasound transducer array 3, potentially codifying prior knowledge on the acceptable physiological range of shape variation.

2. In a step 92, ultrasound scanning sequence is provided that uses sub-apertures from the whole transducer array 3 to acquire images.

3. In a step 93, image registration algorithm is performed that uses local metrics of agreement to assess, whether the different images are properly aligned.

The estimated probe shape can either be iteratively updated or exhaustively sought, based on the local metrics of a sub-aperture image agreement.

The resulting aligned images are then compounded for the estimated probe shape, thereby presenting the best local agreement across the individual sub-aperture images.

One key aspect behind this concept of the method is that the parametric shape model is used to constrain the image registration algorithm, thus ensuring that the alignment of the sub-aperture images respects the physiological range of the anatomical surface to which the probe is adhered to.

Moreover, the method greatly reduces the degrees of freedom of the overall problem, contributing to its efficient and robust implementation.

Regarding the algorithm, for the current estimate of the probe shape, the sub-aperture images are distributed spatially considering the estimated location and orientation of the individual transducer elements 3a. A metric of local agreement (of which an embodiment could be the standard deviation of the samples of all the aligned images within a local region) is then calculated for all the image positions, therefore quantifying if the content of all sub-aperture images is locally similar or not. This approach is demonstrated in **Fig. 9E.**

The probe shape, i.e., the shape of the transducer array 3, is then updated iteratively towards the optimization of the local agreement metric or the entire shape space is exhaustively sought for the probe shape leading to the best agreement.

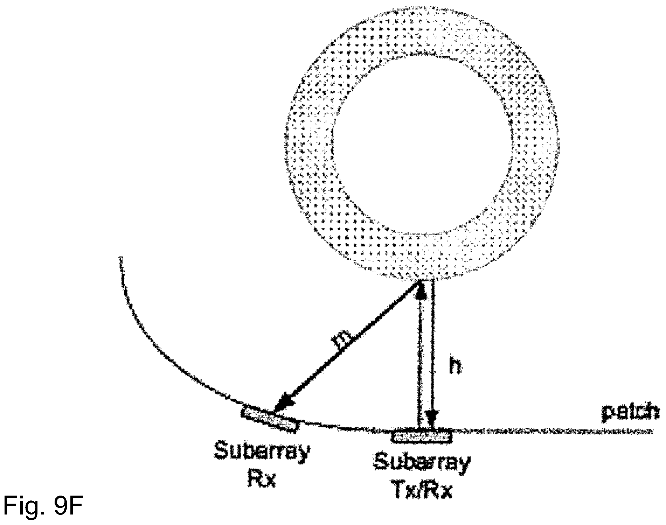

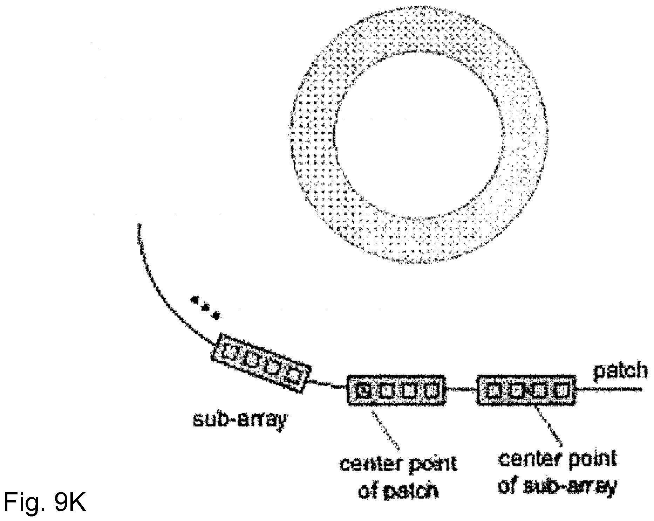

Turning to **Fig. 9F** and **Fig. 9K,** further embodiments of methods for shape calibration are described.

In one embodiment, a time-of-flight calibration is carried out, as demonstrated in **Fig. 9F** to **Fig. 9H.**

For example, at the starting point of the method, a curved patch 2 with the transducer array 3, and a ring-like collection of scatterers, i.e., the scene, in the sample is assumed. This collection of scatterers could, e.g., represent the latitude slice of the left ventricle of a subject.

The curvature of the patch 2, i.e., the curvature or real shape of the transducer array 3, can be estimated by measuring distances h and m, as depicted in **Fig. 9F.** This can be accomplished by measuring time-delays of the reflected signals. This is accomplished in two steps:

In a first step, illustrated by Fig. 9F, a transmit (Tx) sub-array is activated and shoots an ultrasound signal aimed at a reference point in the scene. Beam-steering is used to point the ultrasound signal towards a specific position inside the sample.

The reflection of this ultrasound signal is received by a receive (Rx) sub-array. Beamsteering is used to make sure that the receive sub-array Rx receives the reflected signal from the same location inside the sample as where the transmit sub-array Tx pointed the signal. The correct steering angle for the Rx sub-array can for example be found by performing pulse-echo measurements where the Tx-subarray is sending the pulses and the Rx subarray is receiving the echo's. Varying the Rx angle will lead to a maximum in the receive signal intensity, when the Tx and Rx sub-array focal points are at coinciding or if the send and receive beam-profiles have maximum overlap over the boundary of interest. The steering angle at which the maximum intensity of the signal is achieved give the correct steering angle for the Rx subarray and leads then to the calculation of m and h.

With the data acquired by this procedure, it is possible to calculate the distance h.

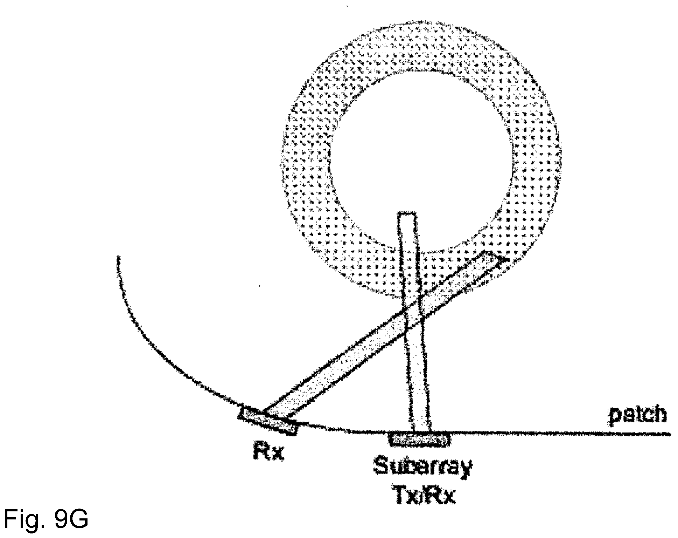

In a second step, illustrated by Fig. 9G, a transmit (Tx) sub-array is activated and shoots an ultrasound signal at a reference point in the scene, wherein beam-steering is used again to direct the ultrasound pulse.

The reflection of this ultrasound pulse is received by a receive (Rx) sub-array, which is directed to a different location as the Tx sub-array by beam-forming.

With the data acquired by this procedure, it is possible to calculate the distance m.

With the information of h and m, relative position between two points in space are calculated, in particular the relative positioning of the transmit Tx and receive Rx sub-arrays of the transducer array 3.

By repeating the procedure for several points along the transducer array 3, the whole shape of the transducer array 3 can be estimated.

This two-step procedure is simple to implement.

However, the following considerations need to be made, when applying the method to ultrasound signals:
One challenge is due to the fact that a transmitted ultrasound wave is reflected not only from the reference point, but from all the neighboring points as well. This leads to a received signal that is the sum of contributions from several different points on the scene.

In the midst of all these different contributing points, it can be difficult to pinpoint the signal, which was actually reflected from the reference point.

This effect is similar to the effect of multi-path configurations for communication systems and can lead to incorrect estimates of h and m.

Another challenge is due to the fact that the reference point is not known a priori. It is thus difficult to adapt the Rx beam-forming delays such as to focus precisely on the reference point.

These challenges may be addressed as follows:
First, several Tx/Rx events are considered. Ideally, the beam-forming gains (or beams) of the Tx and Rx should intersect with the reference point. The intersection should occur not at the center of the beams. Instead, the optimal intersection is the one in which both beams at half of the maximum. This is illustrated in **Fig. 9G.**

In **Fig. 9G,** the width of the beams corresponds to the full width at half maximum.

The intersection of the beams looks like scissors cutting the left ventricle at the reference point. The beams are intersected like this so as to emphasize the signal reflected from the reference point and de-emphasize the signal reflected from all other points. Because the position of the reference point is not known a priori, a sectorial scan should be done on the Rx side.

Second, a L1 minimization problem is solved. To understand why, consider the procedure for the measurement of m. Given the scissor-like beams, the signal obtained by the Rx sub-array should ideally contain big contributions only from a limited number of scatterers. The scatterer distribution is thus sparse. A L1 minimization problem finds the position of these scatterers.

As a result, the full procedure is as follows:

    a. Measure h

        i. transmit and receive at the same sub-array location. Tilt the Tx beam by -x degrees and the Rx beam by x degrees.

        ii. Solve L1 minimization problem and find the biggest scatterer's position

        iii. Calculate h

    b. Measure m

        i. transmit and receive at the different sub-array locations. Tilt the Tx beam by -x degrees and do a sectorial scan on the Rx beam such that it points to several directions

        ii. For each direction solve the L1 minimization problem and find the biggest scatterers position

iii. Calculate m for all directions, discard outliers

c. With m and h, find the relative position of the Rx sub-array by trigonometry.

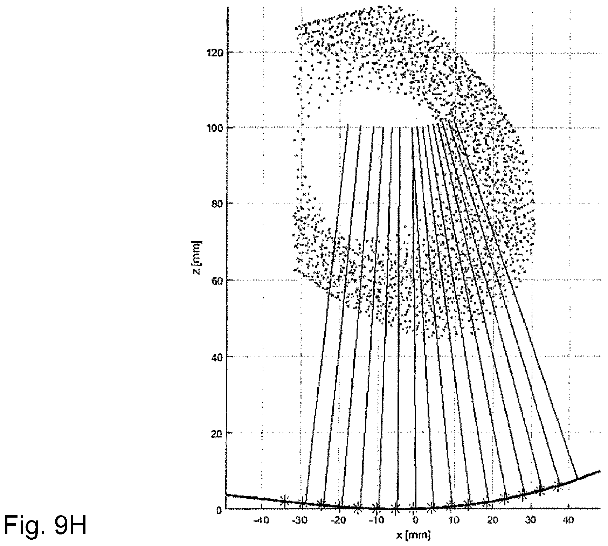

In **Fig. 9H,** results for the estimation of the curvature using time-of-flight calibration are shown.

Further approaches for embodiments of the method comprise the following aspects:
Principal component analysis (PCA) may be used to restrict the search space for the curvature of the transducer array. This aspect is described in further detail above with reference to **Fig. 9D** and **Fig. 9E.**

The window of the L1 minimization can be adapted such that it is narrow and it captures the reference scatterer. This can be done by focusing on the part of the reconstructed signal with the best match to the real signal.

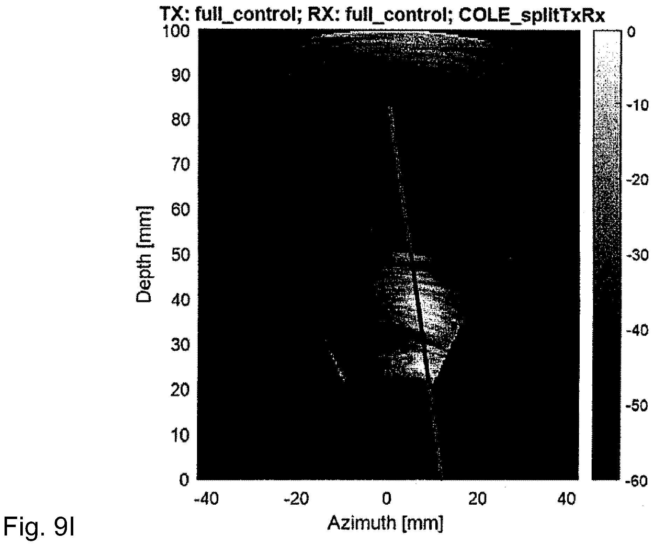

When doing the sectorial scan on the Rx side, the image may be looked at to detect the Tx beam. This gives information on the relative inclination between Tx and Rx sub-arrays. A line can be fitted to the most likely position of the beam. This is exemplary illustrated in **Fig. 9I**.

The curvature of the patch can lead to two types of distortion, depending on how the patch is operated.

Before proceeding, a hierarchy is defined that is applicable to the respective embodiment of the ultrasound system 1. In this hierarchy, the lowest unit considered is the pMUT sub-element 3b (the sensor). Several pMUT sub-elements 3b make a transducer array element 3a. For example, a 3x3 array of pMUT sub-elements 3b form a transducer array element 3a. Several transducer array elements 3a, for example 16x16, make up a sub-array 4. All the sub-arrays 4 make up a transducer array 3, or the "patch".

For the following, the curvature on a sub-array basis is deemed negligible.

The following two types of distortion are defined:
Beam-forming distortion occurs, when there is a mismatch between the calculated and the optimal delays applied to the transducer array elements 3a, which is possible both in the send as well as receive beamforming step. Such beam-forming distortion could occur e.g. when transducer array 3 is driven through the individual transducer array elements 3a, as could be done e.g. with synthetic aperture imaging. Or when the whole array (or sub-array) is driven as a phased array.

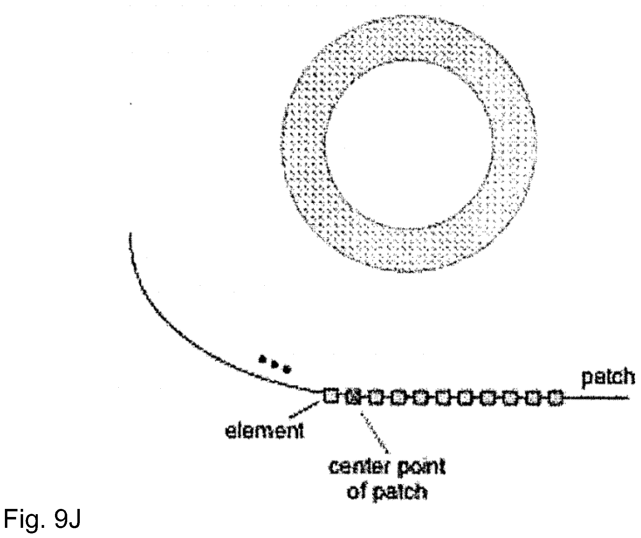

Reference is made to **Fig. 9J.**

All delays are calculated with reference to a center point, which is also shown in figure 9J. If delays are calculated considering a flat transducer array 3, while the transducer array 3 is in reality not flat, then it is clear where the mismatch comes from.

Beam-forming distortion creates a wider point-spread function (PSF). This is akin to a defocusing effect which could happen in an optical device.

On the other hand, geometric distortion occurs, when the transducer array 3 is driven through the sub-arrays 4.

All delays on each sub-array 4 are calculated with reference to the center of the subarray 4, depicted in **Fig. 9K.**

Given that the curvature on the sub-array level is negligible, there is no beam-forming distortion.

Geometric distortion is observed, when combining the information from different sub-arrays 4 by shifts and rotations.

If, when shifting and rotating the shots from sub-arrays 4 so as to compose a final image, a flat transducer array 3 is considered while in reality it is not, an expansion/compression is observed in the image domain.

This is the geometric distortion.

The minimization of the standard deviation of the envelope works extremely well for the beam-forming distortion. With this minimization, it is possible to very accurately estimate the curvature of the transducer array 3. The main aspect of the following description of the embodiment is to show that it is possible to adapt it a specific system 1 configuration, where the transducer array 3 is driven through the sub-arrays 4.

Consider **Fig. 9K** and how the delays are set for each sub-array 4. The delays should be calculated in reference to a center point - not the center point of the sub-arrays 4, but the center point of the center points of the transducer array 3. In this way all delays for a first sub-array 4 should be close to a delay value d1, all delays of sub-array 2 should be close to a delay value d2, and etc.

The delays of an n-th sub-array 4 should follow a triangular pattern with average delay value dn.

In the current system 1, transmitting and receiving is only performed with one subarray 4 at a time. It is therefore necessary to provide a sequence that activates/deactivates Tx and Rx sub-arrays in turn. The signals should all be collected and offline beam-forming would be performed. If, as shown in figure 9K, three sub-arrays are involved, nine Tx/Rx pairs need to be activated in turn.

By using this procedure, the final image may have the beam-forming distortion. Hence the above-described minimization of the standard deviation of the envelope can be used as a metric to find the shape of the transducer array 3.

In connection with the above disclosure, the following aspects are explicitly disclosed:

**Aspect 1:** Ultrasound system (1), in particular for cardiac monitoring, comprising:

- a transducer array (3) with a plurality of transducer array elements (3a);

- a control unit (40), which is coupled to the transducer array (3), for controlling the transducer array (3); and

- a data processing unit (41) for receiving data from the transducer array (3) and processing the received data;

wherein the ultrasound system (1) further comprises a deformation correction module (43), which is configured to perform a deformation correction step, wherein deformation information is determined for the transducer array (3).

**Aspect 2:** Ultrasound system (1) according to Aspect 1, wherein at least one sub-array (4) of transducer array elements (3a) is defined and controlled as a functional unit for sending ultrasound pulses and/or receiving ultrasound signals.

**Aspect 3:** Ultrasound system (1) according to Aspect 1 or Aspect 2, wherein the deformation correction step comprises:

- generating an ultrasound pulse by at least one transducer array element (3a);

- receiving an ultrasound signal by the same and/or another transducer array element (3a);

- optionally determining the deformation information based on the generated ultrasound pulse and the received ultrasound signal,

- determining the deformation correction information either based on the deformation information or directly based on the received ultrasound signals or images derived from the signals,

- correcting the ultrasound signals and/or images based on the deformation correction information.

**Aspect 4:** Ultrasound system (1) according to one of the preceding Aspects, wherein the deformation information comprises positional information for at least one first transducer array element.

**Aspect 5:** Ultrasound system (1) according to one of the preceding Aspects, wherein the deformation correction step further comprises the step of comparing of ultrasound signal or image metrics for self-shape estimation for the transducer array (3). **Aspect 6:** Ultrasound system (1) according to one of the preceding Aspects, wherein the deformation correction step further comprises the step of performing a shapeindependent image correction for the transducer array.

**Aspect 7:** Ultrasound system (1) according to one of the preceding Aspects, wherein the deformation correction step further comprises the following steps:

- determining an intrinsic image reconstruction artifact; and

- performing a shape estimation for the transducer array (3) based on the intrinsic image reconstruction artifact.

**Aspect** 8: Ultrasound system (1) according to one of the preceding Aspects, wherein the deformation correction step further comprises:

- directly filtering the image data acquired with several unknown probe shapes, to directly estimate the true image data without directly recovering the deformation information.

**Aspect 9:** Ultrasound system (1) according to one of the preceding Aspects, wherein the deformation correction step further comprises the following steps:

- generating an ultrasound pulse;

- performing a beamsteering control for receiving a reflected of the ultrasound pulse under at least two angles and generating angle-dependent reflection information; and

- determining the deformation information based on the angle-dependent reflection information.

**Aspect 10:** Ultrasound system (1) according to one of the preceding Aspects, wherein the deformation correction step further comprises the step of performing a delay-based calibration and/or a time-of-flight calibration.

**Aspect 11:** Method for operating an ultrasound system (1) comprising a transducer array (3), especially an ultrasound system (1) according to one of the preceding Aspects, the method comprising at least the step that a deformation correction step is performed, wherein deformation information is determined for the transducer array (3). **Aspect 12:** The method of Aspect 11, wherein a principal component analysis (PCA) is used to perform a sectorial image registration for ultrasound self-shape estimation and image compounding.

**Aspect 13:** The method of Aspect 12, wherein the method comprises at least the following steps:

a) A parametric model is provided, representing the shape of the ultrasound transducer array;

b) An ultrasound scanning sequence is provided that uses sub-apertures from the whole transducer array to acquire images; and

c) An image registration algorithm is executed, wherein local metrics of agreement are used to assess, whether the different images are properly aligned.

**Aspect 14:** The method of one of Aspects 11 to 13, wherein the method comprises the step of a time-of-flight calibration.

**Aspect 15:** The method of Aspect 14, wherein the method comprises at least one or more the following steps:

a) A distance between a transmitting transducer sub-array and a scatterer is measured by transmitting and receiving an ultrasound signal at the same subarray location, wherein further beam-forming is used to tilt a transmitted ultrasound beam (Tx) by a certain angle (-x degrees) and to tilt a received ultrasound beam (Rx) by another certain angle (x degrees), and, subsequently, an L1 minimization problem is solved to determine the biggest scatterer's position, and the distance is calculated;

b) A distance between a receiving transducer sub-array and the scatterer is measured by transmitting and receiving another ultrasound signal at different sub-array locations, wherein further the transmitted ultrasound beam (Tx) is tilted by a certain angle (-x degrees), and a sectorial scan is done on the receiving ultrasound beam (Rx) such that it points to several directions, and where for each direction, the L1 minimization problem is solved to determine the biggest scatterer's position, and the distance is calculated and this distance is calculated for a plurality of directions, and outlier measurements are discarded; and

c) Based on the distances of steps a) and b), a relative position is determined for the receiving (Rx) sub-array by trigonometry.

## Reference numerals

| | |
|---|---|
| 1 | Ultrasound system |
| 2 | Adhesive patch |
| 3 | transducer array; pMUT array |
| 3a | transducer array element; pMUT element; transducer array unit |
| 3b | sub-element; pMUT sub-element |
| 3c | transducer array set |
| 4 | sub-array |
| 5 | thin-film transistor (TFT) array |
| 6 | TFT driver module |
| 7 | analog-digital converter (ADC) |
| 8 | pulser module |
| 9 | digital application-specific integrated circuit (ASIC) |

| | |
|---|---|
| 10 | Analog Front-End |
| 11 | interface module |
| 11a | interface control module ASIC |
| 12 | external unit; back-end unit |
| 13 | memory module (RAM) |
| 14 | power management module |
| 20 | system |
| 21 | transducer array |
| 22 | analog signals |
| 23 | switch driver unit |
| 24 | receiver unit |
| 25 | pulser unit |
| 26 | signal processing; control unit |
| 27 | control module |
| 30 | micro beam-former |
| 31 | analog output channel |
| 32 | delay unit |
| 33 | sum unit |
| 34 | analog-digital converter (ADC) |
| 35 | digital output channel |
| 40 | control unit |
| 41 | data processing unit |
| 42 | complexity reduction module |
| 43 | deformation correction module |
| 44 | correction module |
| 45 | reference module |
| 46 | volume reconstruction module |
| 47 | selected monitoring unit |
| 48 | artifact determination module |
| 49 | phase determination module |
| 51 | step |
| 52 | step |
| 61 | step |
| 62 | step |
| 63 | step |
| 64 | step |
| 71 | step |
| 72 | step |
| 73 | step |
| 74 | step |
| 75 | step |
| 76 | step |
| 77 | step |
| 81 | step |
| 82 | step |
| 83 | step |
| 84 | step |
| 91 | step |
| 92 | step |
| 93 | step |
| 201 | system |
| 202 | patch case |
| 203 | transducer array |
| 205 | TFT array |
| 206 | beam-former module |
| 209 | control device |
| 210 | flexible PCB |
| 211 | USB connector |

211a USB transceiver
212 PC
213 RAM module
214 power management IC
301 system
302 flexible packaging; patch case
303a sensor unit
306 ASIC (AFE) element
309 control device
310 flexible PCB
311 wireless transceiver
313 memory module
314 battery gauge
314a wireless/wired charger
315 power IC
401 system
403 sensor
404 interconnects
406 periphery
406a ICs
410 flexible PCB
414 flexible battery
420 acoustic matching and glue layer
N number of rows
M number of columns

**Claims**

1. Ultrasound system (1), in particular for cardiac monitoring, comprising

   - a transducer array (3) with a plurality of transducer array elements (3a), said transducer array elements (3a) being configured to send ultrasound pulses and/or receive ultrasound signals;
   - a control unit (40), which is coupled to the transducer array (3), for controlling the transducer array (3); and
   - a data processing unit (41) for receiving data from the transducer array (3) and processing the received data, and
   - a deformation correction module (43), which is configured to perform a deformation correction step based on deformation information determined for the transducer array (3),

     wherein the transducer array (3) is a 2-dimensional transducer array, and
     wherein the system (1) further comprises a beam-former module configured to perform a step of applying a predefined time delay to at least one transducer array element (3a) or a plurality of transducer array elements (3a).

2. Ultrasound system (1) according claim 1,
   **characterized in that**
   the predefined time delay is determined by the beam-former module.

3. Ultrasound system (1) according claim 2,
   **characterized in that**

   the predefined time delay is determined for each transducer array element (3a) or plurality of transducer array elements (3a) to which said time delay is to be applied, preferably wherein the beam-former module comprises an electronic delay and sum circuit, configured to apply different time delays to a predefined number of transducer array elements (3a),
   preferably wherein said predefined number of transducer array elements (3a) is comprised between 8 and 32.

4. Ultrasound system (1) according to any of the preceding claims,
   **characterized in that**
   the step of applying a predefined time delay is implemented by the beam-former module when said transducer array element (3a) or plurality of transducer array elements (3a) is/are used for sending ultrasound pulses and/or receiving ultrasound signals.

5. Ultrasound system (1) according to one of the preceding claims,
   **characterized in that**
   wherein at least one sub-array (4) of transducer array elements (3a) is defined and controlled as a functional unit for sending ultrasound pulses and/or receiving ultrasound signals.

6. Ultrasound system (1) according to claim 5,
   **characterized in that**
   said at least one sub-array (4) is dynamically defined and capable of changing its position.

7. Ultrasound system (1) according to one of the preceding claims,
   **characterized in that**
   the deformation correction step comprises:

   - generating an ultrasound pulse by at least one transducer array element (3a);
   - receiving an ultrasound signal by the same and/or another transducer array element (3a);
   - optionally determining the deformation information based on the generated ultrasound pulse and the received ultrasound signal,
   - determining the deformation correction information either based on the deformation information or directly based on the received ultrasound signals or images derived from the signals,
   - correcting the ultrasound signals and/or images based on the deformation correction information.

8. Ultrasound system (1) according to one of the preceding claims,
   **characterized in that**
   the deformation information comprises positional information for at least one first transducer array element (3a).

9. Ultrasound system (1) according to one of the preceding claims,
   **characterized in that**
   the deformation correction step further comprises the step of comparing of ultrasound signal or image metrics for self-shape estimation for the transducer array (3).

10. Ultrasound system (1) according to one of the preceding claims,
    **characterized in that**
    the deformation correction step further comprises the step of performing a shapeindependent image correction for the transducer array.

11. Ultrasound system (1) according to one of the preceding claims,
    **characterized in that**
    the deformation correction step further comprises the following steps of:

    - determining an intrinsic image reconstruction artifact; and
    - performing a shape estimation for the transducer array (3) based on the intrinsic image reconstruction artifact.

12. Ultrasound system (1) according to one of the preceding claims,
    **characterized in that**
    the deformation correction step further comprises:

    - directly filtering the image data acquired with several unknown probe shapes, to directly estimate the true image data without directly recovering the deformation information.

13. Ultrasound system (1) according to one of the preceding claims,
    **characterized in that**
    the deformation correction step further comprises the following steps:

    - generating an ultrasound pulse;
    - performing a beamsteering control for receiving a reflected signal of the ultrasound pulse under at least two angles and generating angle-dependent reflection information; and
- determining the deformation information based on the angle-dependent reflection information.

14. Ultrasound system (1) according to one of the preceding claims,
    **characterized in that**
    the deformation correction step further comprises the step of performing a delay-based calibration and/or a time-of-flight calibration.

15. Ultrasound system (1) according to one of the preceding claims,
    **characterized in that**
    wherein a plurality of sub-arrays (4) is defined in the transducer array (3), said plurality of sub-arrays (4) being arranged such that no overlap occurs between array elements (3a) of different sub-arrays (4).

Transducer array = sensor array + switch (TFT) array

Analog signals

Switch Driver

Receiver

Pulser

Signal processing, Control

Ultrasound algorithms, Data Analysis, Visualization

Control

Fig. 1

Fig. 2A

EP 4 529 857 A2

Fig. 2B

Fig. 2C

Fig. 2D

EP 4 529 857 A2

Fig. 3A

Fig. 3B

Fig. 3C

Fig. 3D

EP 4 529 857 A2

EP 4 529 857 A2

Fig. 4

Fig. 5

Fig. 6

Fig. 7

Fig. 8

Fig. 9A

Fig. 9B

Fig. 9C

Fig. 9D

**True probe shape**

*Physiological range of acceptable shapes*

**Good probe shape estimate**

**Good sub-aperture image alignment**

**Poor probe shape estimate**

**Poor sub-aperture image alignment**

Fig. 9E

Fig. 9F

Fig. 9G

Fig. 9H

TX: full_control; RX: full_control; COLE_splitTxRx

Fig. 9I

EP 4 529 857 A2

Fig. 9J

Fig. 9K

sub-array

patch

center point
of patch

center point
of sub-array

**REFERENCES CITED IN THE DESCRIPTION**

**Patent documents cited in the description**

- US 20160136462 A1
- WO 2018102828 A1

- WO 2019162485 A1

**Non-patent literature cited in the description**

- **GERARDO et al.** Fabrication and testing of polymer-based capacitive micro-machined ultrasound transducers for medical imaging. *Microsystems & Nanoengineering*, 2018, vol. 4, 19